# EUROPEAN PATENT APPLICATION

(11) **EP 1 293 571 A2**
(43) Date of publication of application: **19.03.2003**
(21) Application number: 02080082.7
(22) Date of filing: 07.07.1993
(51) Int. Cl.: C12N 15/62, C12N 11/16, C12N 15/56, C12N 15/55, C12N 15/53, C12N 9/04, C12N 9/20, C12N 9/40

(54) **Process for immobilizing enzymes to the cell wall of a microbial cell by producing a fusion protein**

(30) Priority: 08.07.1992 EP 92202080
(62) Divisional of application: 93915827.5
(71) Applicant: UNILEVER N.V., 3013 AL Rotterdam (NL); UNILEVER PLC, London EC4P 4BQ (GB)
(72) Inventor: Klis, Franciscus Maria, c/o Unilever R & D, 3133 AT Vlaardingen (NL); Schreuder, Maarten Pleun, c/o Unilever R & D, 3133 AT Vlaardingen (NL); Toschka, Holger York, c/o Unilever R & D, 3133 AT Vlaardingen (NL); Verrips, Cornelis Theodorus, c/o Unilever R & D., 3133 AT Vlaardingen (NL)

(57) **Abstract**

A method is provided for immobilizing an enzyme, comprising immobilizing the enzyme or a functional part thereof to the cell wall of a microbial cell using recombinant DNA techniques. The enzyme is immobilized by linking it to the C-terminal part of a protein that ensures anchoring in the cell wall.
Also provided is a recombinant polynucleotide comprising a structural gene encoding an enzyme protein, a part of a gene encoding the C-terminal part of a protein capable of anchoring in a eukaryotic or prokaryotic cell wall, as well as a signal sequence, in addition to a chimeric protein encoded by the recombinant polynucleotide and a vector and a microorganism containing the polynucleotide. The microorganism is suitable for carrying out enzymatic processes on an industrial scale.

SEQUENCE LISTING

(1) GENERAL INFORMATION:

(i) APPLICANT:
(A) NAME: Unilever N.V.
(B) STREET: Weena 455
(C) CITY: Rotterdam
(E) COUNTRY: The Netherlands
(F) POSTAL CODE (ZIP): NL-3013 AL

(A) NAME: Unilever PLC
(B) STREET: Unilever House Blackfriars
(C) CITY: London
(E) COUNTRY: United Kingdom
(F) POSTAL CODE (ZIP): EC4P 4BQ

(A) NAME: Franciscus Maria KLIS
(B) STREET: Benedenlangs 102
(C) CITY: Amsterdam
(E) COUNTRY: The Netherlands
(F) POSTAL CODE (ZIP): NL-1025 KL

(A) NAME: Maarten Pleun SCHREUDER
(B) STREET: Rode Kruislaan 1220
(C) CITY: Diemen
(E) COUNTRY: The Netherlands
(F) POSTAL CODE (ZIP): NL-1111 XB

(A) NAME: Holger York TOSCHKA
(B) STREET: Coornhertstraat 77
(C) CITY: Vlaardingen
(E) COUNTRY: The Netherlands
(F) POSTAL CODE (ZIP): NL-3132 GB

(A) NAME: Cornelis Theodorus VERRIPS
(B) STREET: Hagedoorn 18
(C) CITY: Maassluis
(E) COUNTRY: The Netherlands
(F) POSTAL CODE (ZIP): NL-3142 KB

(ii) TITLE OF INVENTION: Enzymic Processes based on naturally
immobilized enzymes that can easily be separated and
regenerated

(iii) NUMBER OF SEQUENCES: 26

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 6057 base pairs
(B) TYPE: nucleic acid
(C) STRANDEDNESS: double
(D) TOPOLOGY: linear

(ii) MOLECULE TYPE: DNA (genomic)

(vi) ORIGINAL SOURCE:
(A) ORGANISM: Saccharomyces cerevisiae

(ix) FEATURE:
(A) NAME/KEY: CDS
(B) LOCATION: 3653..5605
(D) OTHER INFORMATION: /function= "sexual agglutinisation"
/product= "alpha-agglutinin"

(xi) SEQUENCE DESCRIPTION: SEQ ID NO: 1:

AAGCTTTAGG TAAGGGAGGC AGGGGGAAAA GATACTGAAA TGACGGAAAA CGAGAATATG 60

GAGCAGGGAG CAACTTTTAG AGCTTTACCC GTTAAAAGGT CAAATCGAGG CTTCCTGCCT 120

TTGTCTGATT TTAGTAGTAC CGGAAGGTTT ATTACGCCCA AGAACAGTGC TTGAATTGAG 180

TTCTCGGGAC ACGGGAAAGA CAATGGAAGA AAAATTTACA TTCAGTAGCC TTATATATGA 240

AATGCTGCCA AGCCACGTCT TTATAAGTAG ATAATGTCCC ATGAGCTGAA CTATGGGAAT 300

TTATGACGCA GTTCATTGTA TATATATTAC ATTAACTCTT TAGTTTAACA TCTGAATTGT 360

TTTATAAAAT AACTTTTTGA ATTTTTTTAT GATCGCTTAG TTAAGTCTAT TATATCAGGT 420

TTTTTCATTC ATCATAATTG TTCGTTAAAT ATGAGTATAT TTAAATACAG GAATTAGTAT 480

CATTTGCAGT CACGAAAAGG GCCGTTTCAT AGAGAGTTTT CTTAATAAAG TTGAGGGTTT 540

CCGTGATAGT TTTGAGGGGT TGTTTGAACT AGATTTACGC TTACCTTTCA ACTGATTAAT 600

TTTTTCAGCG GGCTTATCAT AATCATCCAT CATAGCAGTC TTTCTGGACT TCGTCGAGGA 660

CTGGCTTTCT GAATTTTGAC GGTCCCTATT AGCTCCAGTT GGAGGAATTG AGTTACCTAC 720

AACTGGCAAG AGGTCTTTGT TTGGATTCAA AATAGGACTT TGTGGTAGCA GTTTGGTTTT 780

ATTCAATCTA AAGATATGAG AAACAGGTTT TAAGTAAATC GATACTATTG TACCAATGTT 840

TAGCTCCAAT TCCTCCAAAA CGGTGGGATC TAATTTTGTG TTCATTTCTA TTAGTGGCAA 900

CTCTCCGTCC AGTACTGATT TTAAAGATTC AAAAGTTATC GCGTTTGATA TACGAGACGT 960

TTTCGTTAAT GACAGCAATC TCCAATACAT CAGTGTTTTA TCTCTTAAGT CAGGATTATT 1020

TTCGTGATCG GTGCATCCTT TTAATAAATC CATACAAAGT TCTTCAGTTT CCTTTGTAGG 1080

ATTTCTGATG AAGAATTTTA TTGCTGAGTT CAGAATGGAA AATTGCACTT CTAGCGTCTC 1140

ATTAAACATG TTTGAGGAAA AAACTCTAAA TAACTCCAGG TAGTTTGGAA TTACATCCGA 1200

ATATTGCGTT ATTATCCAGA TCATAGCGTT TTTTGATTCA GGTTCCTGTA CAACTTCAGT 1260

GTGTTTGACT AGTTCTGTTA CGTTTGCTTT AAAATTATTG GGATATTTCC TCAAAATATT 1320

TCTGAAAACC GAAATAATCT CCTGGACGAC ATAATCAACA CCGAATTCTA ACAAATCTAG 1380

TAGCACAGCG ACACAATCGT GTACAGAGTC TTCATCTAGC TTAACAGCGA GATTACCAAT 1440

GGCTCTGACT GATTTCCTTG ACATTTGAAT ATCAATATCT GTAGCATATT GTTCCAACTC 1500

TTCTAGAATT CTTGGTAATG TTTCCTTGTT AGCTAAAAGA TATAAACACT CTAATTTCGT 1560

GTCTTTGATG TATATGGGGT CATTGTACTC GATGAAAAAA TACGAAATGT CTAGCCTGAG 1620

TAGAGATGAC TCCCTACTCA ATAAAAGAAG AATAACGTTT CTTAATACTA AAAATTGTAA 1680

TTCAGGCGGC TTATCTAACA AAGCTATTAC AGAGTTAGAT AGCTTTTCGG CTAGAGTTTC 1740

TTTGATGACG TCAACATAAT TCAACAAGTA CATGATGAAT TTTAAAGAGT TCAACACTAC 1800

GTATGTGTTT ACTTGTTGCA GGTACGGTAA AGCTAGTTCG ATCATTTCAT GGGTATCCAA 1860

ATAATGCTGC GGCACAACCG AAGTCGTCAA AACTTCCAAA ACAGTAGCCT TATTCCACTC 1920

ATTTAATTCG GGTAAAAGTT CTAGCATGTC AAAAGCGAGT TCCAAGGGAA TCCTGAAGGT 1980

TCCATGTTAG CGTTTTTTTC GTGAATGGAA TATAAAGTAT GTAATGCAGC TACAATGACT 2040

TCTGGAGAGC TCGACTGTGC CTTTACAATG TCATGTAGAA TGCTTGATAA CCCCAATACC 2100

CTTTCATGAT CAATTTCATC TAAATCCAAC AGTGCGTAAA TTGCTGTCCT CGTCACTTGT 2160

TCAGGTGGAG ACTTGTGATT TACCAATGAA ATGATACAGT CGAAGGCCTG ATCAGATAGC 2220

TCTTTCACCG GGACTAATAC CAGAGTTCTT AGTGCCATTA TTTGTAACTT TTCATCTCTG 2280

CTTTTGAAAT CGTCCATTAT AAATGGCAAA GCCTCTCTGG CCTGCTGAGG TTTTAATGCG 2340

CCGATCACCC TAATATACTC ATGGCAAATT CTTTTCACTT CTAGATCATC TTCAATTTGC 2400

CAAAATTTCA AGAGCTCAGA AAACAGAAGG GACATTTCGC CATAGTTTCC TAGAACCAAA 2460

TTGGCGATAA TTTTTCTCAG AGCATTTTTC CTTCTTGTTA TATTCGATTT AAACTTTTTT 2520

ACTCCAAAAT GTTGCAGATC TGTGACGATT TCATTTGCTT TATATCTGGC AAAAACTTTT 2580

TGATCGGACA TAAGCGAAAT ACGTCCTATT AATGAAGTGA ATGTTCTTGC TGTATTCCCT 2640

TCTTGTGCAG TAGATTAATT CTGTTTCCAG GCTGCGATAC TTTGATACCC AATACTAAAA 2700

GTTGATGATT TGAACGATCT CCTATTTCCT CGCACATTTT TGGAGCGATA CCCGGAAGAC 2760

AGAATCGCGA TGTTAAGAAA ATAGTTCTGA TGGCACTAAA GAGATCATGA TTAAGGAAAG 2820

GTAAGTGATA TGCATGAATG GGAATAGGCT TTCGAACTTG ACGATTTAGT TCCTTATTTC 2880

TATCCATCTA ATCCTCCAAC TTCAATAGGC CTTATCTAGC TCAGAGCAGT ATTTAATTGA 2940

GAATAGTAGC TTAATTGAAA CCTTACTAAA AAAGTGTATG GTTACATAAG ATAAGGCGTT 3000

AAGAAGAGTA TACATATGCA TTATTCATTA CCAAGACCAC TATGAATAGT AATACCATAT 3060

TTAGCTTTTG AAACTCATGT TTTCTATTGT GTTGTTTCAA ATTCCTCTGT TAGGCTCAAT 3120

TTAGGTTAAT TAAATTATAA AAAAATATAA AAAATAAAGA AAGTTTATCC ATCGGCACCT 3180

CAATTCAATG GAGTAAACAG TTTCAACACT GAGTGGTGAA ACATTGAACA ACTACATGCA 3240

GTTTCCCGCC ACGAGGCAAG TGTAGGTCCT TTGTCCATTT CGCTTTGTTT TGCAGGTCAT 3300

TGATGACCTA ATTAGGAAGG TAGAAGCCGC TCCAGCTCAA TAAGGAAATG CTAAGGGTAC 3360

TCGCCTTTGG TGTTTTACCA TACAATGGCA GCTTTATGTC ACTTCATTCT TCAGTAACGG 3420

CGCTTAAATA TTCCCAAAAA CGTTACAATG GAATTGTTTG ATCATGTAAC GAAATGCAAT 3480

CTTCTAAAAA AAAAGCCATG TGAATCAAAA AAAGATTCCT TTTAGCATAC TATAAATATG 3540

CAAAATGCCC TCTATTTATT CTAGTAATCG TCCATTCTCA TATCTTCCTT ATATCAGTCG 3600

CCTCGCTTAA TATAGTCAGC ACAAAAGGAA CAACAATTCG CCAGTTTTCA AA ATG 3655
Met
1

TTC ACT TTT CTC AAA ATT ATT CTG TGG CTT TTT TCC TTG GCA TTG GCC 3703
Phe Thr Phe Leu Lys Ile Ile Leu Trp Leu Phe Ser Leu Ala Leu Ala
            5                   10                  15

TCT GCT ATA AAT ATC AAC GAT ATC ACA TTT TCC AAT TTA GAA ATT ACT 3751
Ser Ala Ile Asn Ile Asn Asp Ile Thr Phe Ser Asn Leu Glu Ile Thr
        20                  25                  30

CCA CTG ACT GCA AAT AAA CAA CCT GAT CAA GGT TGG ACT GCC ACT TTT 3799
Pro Leu Thr Ala Asn Lys Gln Pro Asp Gln Gly Trp Thr Ala Thr Phe
    35                  40                  45

GAT TTT AGT ATT GCA GAT GCG TCT TCC ATT AGG GAG GGC GAT GAA TTC 3847
Asp Phe Ser Ile Ala Asp Ala Ser Ser Ile Arg Glu Gly Asp Glu Phe
50                  55                  60                  65

ACA TTA TCA ATG CCA CAT GTT TAT AGG ATT AAG CTA TTA AAC TCA TCG 3895
Thr Leu Ser Met Pro His Val Tyr Arg Ile Lys Leu Leu Asn Ser Ser
                70                  75                  80

CAA ACA GCT ACT ATT TCC TTA GCG GAT GGT ACT GAG GCT TTC AAA TGC 3943
Gln Thr Ala Thr Ile Ser Leu Ala Asp Gly Thr Glu Ala Phe Lys Cys
            85                  90                  95

TAT GTT TCG CAA CAG GCT GCA TAC TTG TAT GAA AAT ACT ACT TTC ACA 3991
Tyr Val Ser Gln Gln Ala Ala Tyr Leu Tyr Glu Asn Thr Thr Phe Thr
        100                 105                 110

TGT ACT GCT CAA AAT GAC CTG TCC TCC TAT AAT ACG ATT GAT GGA TCC 4039
Cys Thr Ala Gln Asn Asp Leu Ser Ser Tyr Asn Thr Ile Asp Gly Ser
    115                 120                 125

ATA ACA TTT TCG CTA AAT TTT AGT GAT GGT GGT TCC AGC TAT GAA TAT 4087
Ile Thr Phe Ser Leu Asn Phe Ser Asp Gly Gly Ser Ser Tyr Glu Tyr
130                 135                 140                 145

GAG TTA GAA AAC GCT AAG TTT TTC AAA TCT GGG CCA ATG CTT GTT AAA 4135
Glu Leu Glu Asn Ala Lys Phe Phe Lys Ser Gly Pro Met Leu Val Lys
                150                 155                 160

CTT GGT AAT CAA ATG TCA GAT GTG GTG AAT TTC GAT CCT GCT GCT TTT 4183
Leu Gly Asn Gln Met Ser Asp Val Val Asn Phe Asp Pro Ala Ala Phe
            165                 170                 175

ACA GAG AAT GTT TTT CAC TCT GGG CGT TCA ACT GGT TAC GGT TCT TTT 4231
Thr Glu Asn Val Phe His Ser Gly Arg Ser Thr Gly Tyr Gly Ser Phe
        180                 185                 190

GAA AGT TAT CAT TTG GGT ATG TAT TGT CCA AAC GGA TAT TTC CTG GGT 4279
Glu Ser Tyr His Leu Gly Met Tyr Cys Pro Asn Gly Tyr Phe Leu Gly
    195                 200                 205

GGT ACT GAG AAG ATT GAT TAC GAC AGT TCC AAT AAC AAT GTC GAT TTG 4327
Gly Thr Glu Lys Ile Asp Tyr Asp Ser Ser Asn Asn Asn Val Asp Leu
210                 215                 220                 225

GAT TGT TCT TCA GTT CAG GTT TAT TCA TCC AAT GAT TTT AAT GAT TGG 4375
Asp Cys Ser Ser Val Gln Val Tyr Ser Ser Asn Asp Phe Asn Asp Trp
                230                 235                 240

TGG TTC CCG CAA AGT TAC AAT GAT ACC AAT GCT GAC GTC ACT TGT TTT 4423
Trp Phe Pro Gln Ser Tyr Asn Asp Thr Asn Ala Asp Val Thr Cys Phe
            245                 250                 255

GGT AGT AAT CTG TGG ATT ACA CTT GAC GAA AAA CTA TAT GAT GGG GAA 4471
Gly Ser Asn Leu Trp Ile Thr Leu Asp Glu Lys Leu Tyr Asp Gly Glu
        260                 265                 270

ATG TTA TGG GTT AAT GCA TTA CAA TCT CTA CCC GCT AAT GTA AAC ACA 4519
Met Leu Trp Val Asn Ala Leu Gln Ser Leu Pro Ala Asn Val Asn Thr
    275                 280                 285

ATA GAT CAT GCG TTA GAA TTT CAA TAC ACA TGC CTT GAT ACC ATA GCA 4567
Ile Asp His Ala Leu Glu Phe Gln Tyr Thr Cys Leu Asp Thr Ile Ala
290                 295                 300                 305

AAT ACT ACG TAC GCT ACG CAA TTC TCG ACT ACT AGG GAA TTT ATT GTT 4615
Asn Thr Thr Tyr Ala Thr Gln Phe Ser Thr Thr Arg Glu Phe Ile Val
                310                 315                 320

TAT CAG GGT CGG AAC CTC GGT ACA GCT AGC GCC AAA AGC TCT TTT ATC 4663
Tyr Gln Gly Arg Asn Leu Gly Thr Ala Ser Ala Lys Ser Ser Phe Ile
            325                 330                 335

TCA ACC ACT ACT ACT GAT TTA ACA AGT ATA AAC ACT AGT GCG TAT TCC 4711
Ser Thr Thr Thr Thr Asp Leu Thr Ser Ile Asn Thr Ser Ala Tyr Ser
        340                 345                 350

ACT GGA TCC ATT TCC ACA GTA GAA ACA GGC AAT CGA ACT ACA TCA GAA 4759
Thr Gly Ser Ile Ser Thr Val Glu Thr Gly Asn Arg Thr Thr Ser Glu
    355                 360                 365

GTG ATC AGT CAT GTG GTG ACT ACC AGC ACA AAA CTG TCT CCA ACT GCT 4807
Val Ile Ser His Val Val Thr Thr Ser Thr Lys Leu Ser Pro Thr Ala
370                 375                 380                 385

ACT ACC AGC CTG ACA ATT GCA CAA ACC AGT ATC TAT TCT ACT GAC TCA 4855
Thr Thr Ser Leu Thr Ile Ala Gln Thr Ser Ile Tyr Ser Thr Asp Ser
                390                 395                 400

AAT ATC ACA GTA GGA ACA GAT ATT CAC ACC ACA TCA GAA GTG ATT AGT 4903
Asn Ile Thr Val Gly Thr Asp Ile His Thr Thr Ser Glu Val Ile Ser
            405                 410                 415

GAT GTG GAA ACC ATT AGC AGA GAA ACA GCT TCG ACC GTT GTA GCC GCT 4951
Asp Val Glu Thr Ile Ser Arg Glu Thr Ala Ser Thr Val Val Ala Ala
        420                 425                 430

CCA ACC TCA ACA ACT GGA TGG ACA GGC GCT ATG AAT ACT TAC ATC CCG 4999
Pro Thr Ser Thr Thr Gly Trp Thr Gly Ala Met Asn Thr Tyr Ile Pro
    435                 440                 445

CAA TTT ACA TCC TCT TCT TTC GCA ACA ATC AAC AGC ACA CCA ATA ATC 5047
Gln Phe Thr Ser Ser Ser Phe Ala Thr Ile Asn Ser Thr Pro Ile Ile
450                 455                 460                 465

TCT TCA TCA GCA GTA TTT GAA ACC TCA GAT GCT TCA ATT GTC AAT GTG 5095
Ser Ser Ser Ala Val Phe Glu Thr Ser Asp Ala Ser Ile Val Asn Val
                470                 475                 480

CAC ACT GAA AAT ATC ACG AAT ACT GCT GCT GTT CCA TCT GAA GAG CCC 5143
His Thr Glu Asn Ile Thr Asn Thr Ala Ala Val Pro Ser Glu Glu Pro
            485                 490                 495

ACT TTT GTA AAT GCC ACG AGA AAC TCC TTA AAT TCC TTC TGC AGC AGC 5191
Thr Phe Val Asn Ala Thr Arg Asn Ser Leu Asn Ser Phe Cys Ser Ser
        500                 505                 510

AAA CAG CCA TCC AGT CCC TCA TCT TAT ACG TCT TCC CCA CTC GTA TCG 5239
Lys Gln Pro Ser Ser Pro Ser Ser Tyr Thr Ser Ser Pro Leu Val Ser
    515                 520                 525

TCC CTC TCC GTA AGC AAA ACA TTA CTA AGC ACC AGT TTT ACG CCT TCT 5287
Ser Leu Ser Val Ser Lys Thr Leu Leu Ser Thr Ser Phe Thr Pro Ser
530                 535                 540                 545

GTG CCA ACA TCT AAT ACA TAT ATC AAA ACG GAA AAT ACG GGT TAC TTT 5335
Val Pro Thr Ser Asn Thr Tyr Ile Lys Thr Glu Asn Thr Gly Tyr Phe
                550                 555                 560

GAG CAC ACG GCT TTG ACA ACA TCT TCA GTT GGC CTT AAT TCT TTT AGT 5383
Glu His Thr Ala Leu Thr Thr Ser Ser Val Gly Leu Asn Ser Phe Ser
            565                 570                 575

GAA ACA GCA CTC TCA TCT CAG GGA ACG AAA ATT GAC ACC TTT TTA GTG 5431
Glu Thr Ala Leu Ser Ser Gln Gly Thr Lys Ile Asp Thr Phe Leu Val
        580                 585                 590

TCA TCC TTG ATC GCA TAT CCT TCT TCT GCA TCA GGA AGC CAA TTG TCC 5479
Ser Ser Leu Ile Ala Tyr Pro Ser Ser Ala Ser Gly Ser Gln Leu Ser
    595                 600                 605

GGT ATC CAA CAG AAT TTC ACA TCA ACT TCT CTC ATG ATT TCA ACC TAT 5527
Gly Ile Gln Gln Asn Phe Thr Ser Thr Ser Leu Met Ile Ser Thr Tyr
610                 615                 620                 625

GAA GGT AAA GCG TCT ATA TTT TTC TCA GCT GAG CTC GGT TCG ATC ATT 5575
Glu Gly Lys Ala Ser Ile Phe Phe Ser Ala Glu Leu Gly Ser Ile Ile
                630                 635                 640

TTT CTG CTT TTG TCG TAC CTG CTA TTC TAAAACGGGT ACTGTACAGT 5622
Phe Leu Leu Leu Ser Tyr Leu Leu Phe
            645                 650

TAGTACATTG AGTCGAAATA TACGAAATTA TTGTTCATAA TTTTCATCCT GGCTCTTTTT 5682

TTCTTCAACC ATAGTTAAAT GGACAGTTCA TATCTTAAAC TCTAATAATA CTTTTCTAGT 5742

TCTTATCCTT TTCCGTCTCA CCGCAGATTT TATCATAGTA TTAAATTTAT ATTTTGTTCG 5802

TAAAAAGAAA AATTTGTGAG CGTTACCGCT CGTTTCATTA CCCGAAGGCT GTTTCAGTAG 5862

ACCACTGATT AAGTAAGTAG ATGAAAAAAT TTCATCACCA TGAAAGAGTT CGATGAGAGC 5922

TACTTTTTCA AATGCTTAAC AGCTAACCGC CATTCAATAA TGTTACGTTC TCTTCATTCT 5982

GCGGCTACGT TATCTAACAA GAGGTTTTAC TCTCTCATAT CTCATTCAAA TAGAAAGAAC 6042

ATAATCAAAA AGCTT 6057

(2) INFORMATION FOR SEQ ID NO: 2:

(i) SEQUENCE CHARACTERISTICS:
(A) LENGTH: 650 amino acids
(B) TYPE: amino acid
(D) TOPOLOGY: linear

(ii) MOLECULE TYPE: protein

(xi) SEQUENCE DESCRIPTION: SEQ ID NO: 2:

Met Phe Thr Phe Leu Lys Ile Ile Leu Trp Leu Phe Ser Leu Ala Leu
1               5                   10                  15

Ala Ser Ala Ile Asn Ile Asn Asp Ile Thr Phe Ser Asn Leu Glu Ile
            20                  25                  30

Thr Pro Leu Thr Ala Asn Lys Gln Pro Asp Gln Gly Trp Thr Ala Thr
        35                  40                  45

Phe Asp Phe Ser Ile Ala Asp Ala Ser Ser Ile Arg Glu Gly Asp Glu
    50                  55                  60

Phe Thr Leu Ser Met Pro His Val Tyr Arg Ile Lys Leu Leu Asn Ser
65                  70                  75                  80

Ser Gln Thr Ala Thr Ile Ser Leu Ala Asp Gly Thr Glu Ala Phe Lys
                85                  90                  95

Cys Tyr Val Ser Gln Gln Ala Ala Tyr Leu Tyr Glu Asn Thr Thr Phe
            100                 105                 110

Thr Cys Thr Ala Gln Asn Asp Leu Ser Ser Tyr Asn Thr Ile Asp Gly
        115                 120                 125

Ser Ile Thr Phe Ser Leu Asn Phe Ser Asp Gly Gly Ser Ser Tyr Glu
    130                 135                 140

Tyr Glu Leu Glu Asn Ala Lys Phe Phe Lys Ser Gly Pro Met Leu Val
145                 150                 155                 160

Lys Leu Gly Asn Gln Met Ser Asp Val Val Asn Phe Asp Pro Ala Ala
                165                 170                 175

Phe Thr Glu Asn Val Phe His Ser Gly Arg Ser Thr Gly Tyr Gly Ser
            180                 185                 190

Phe Glu Ser Tyr His Leu Gly Met Tyr Cys Pro Asn Gly Tyr Phe Leu
        195                 200                 205

Gly Gly Thr Glu Lys Ile Asp Tyr Asp Ser Ser Asn Asn Asn Val Asp
    210                 215                 220

Leu Asp Cys Ser Ser Val Gln Val Tyr Ser Ser Asn Asp Phe Asn Asp
225                 230                 235                 240

Trp Trp Phe Pro Gln Ser Tyr Asn Asp Thr Asn Ala Asp Val Thr Cys
                245                 250                 255

Phe Gly Ser Asn Leu Trp Ile Thr Leu Asp Glu Lys Leu Tyr Asp Gly
            260                 265                 270

Glu Met Leu Trp Val Asn Ala Leu Gln Ser Leu Pro Ala Asn Val Asn
        275                 280                 285

Thr Ile Asp His Ala Leu Glu Phe Gln Tyr Thr Cys Leu Asp Thr Ile
    290                 295                 300

Ala Asn Thr Thr Tyr Ala Thr Gln Phe Ser Thr Thr Arg Glu Phe Ile
305                 310                 315                 320

Val Tyr Gln Gly Arg Asn Leu Gly Thr Ala Ser Ala Lys Ser Ser Phe
                325                 330                 335

Ile Ser Thr Thr Thr Thr Asp Leu Thr Ser Ile Asn Thr Ser Ala Tyr
            340                 345                 350

Ser Thr Gly Ser Ile Ser Thr Val Glu Thr Gly Asn Arg Thr Thr Ser
        355                 360                 365

Glu Val Ile Ser His Val Val Thr Thr Ser Thr Lys Leu Ser Pro Thr
    370                 375                 380

Ala Thr Thr Ser Leu Thr Ile Ala Gln Thr Ser Ile Tyr Ser Thr Asp
385                 390                 395                 400

Ser Asn Ile Thr Val Gly Thr Asp Ile His Thr Thr Ser Glu Val Ile
                405                 410                 415

Ser Asp Val Glu Thr Ile Ser Arg Glu Thr Ala Ser Thr Val Val Ala
            420                 425                 430

Ala Pro Thr Ser Thr Thr Gly Trp Thr Gly Ala Met Asn Thr Tyr Ile
        435                 440                 445

Pro Gln Phe Thr Ser Ser Ser Phe Ala Thr Ile Asn Ser Thr Pro Ile
    450                 455                 460

Ile Ser Ser Ser Ala Val Phe Glu Thr Ser Asp Ala Ser Ile Val Asn
465                 470                 475                 480

Val His Thr Glu Asn Ile Thr Asn Thr Ala Ala Val Pro Ser Glu Glu
                485                 490                 495

Pro Thr Phe Val Asn Ala Thr Arg Asn Ser Leu Asn Ser Phe Cys Ser
            500                 505                 510

Ser Lys Gln Pro Ser Ser Pro Ser Ser Tyr Thr Ser Ser Pro Leu Val
        515                 520                 525

Ser Ser Leu Ser Val Ser Lys Thr Leu Leu Ser Thr Ser Phe Thr Pro
    530                 535                 540

Ser Val Pro Thr Ser Asn Thr Tyr Ile Lys Thr Glu Asn Thr Gly Tyr
545                 550                 555                 560

Phe Glu His Thr Ala Leu Thr Thr Ser Ser Val Gly Leu Asn Ser Phe
                565                 570                 575

Ser Glu Thr Ala Leu Ser Ser Gln Gly Thr Lys Ile Asp Thr Phe Leu
            580                 585                 590

Val Ser Ser Leu Ile Ala Tyr Pro Ser Ser Ala Ser Gly Ser Gln Leu
        595                 600                 605

Ser Gly Ile Gln Gln Asn Phe Thr Ser Thr Ser Leu Met Ile Ser Thr
    610                 615                 620

Tyr Glu Gly Lys Ala Ser Ile Phe Phe Ser Ala Glu Leu Gly Ser Ile
625                 630                 635                 640

Ile Phe Leu Leu Leu Ser Tyr Leu Leu Phe
                645                 650

(2) INFORMATION FOR SEQ ID NO: 3:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: primer lipol

(xi) SEQUENCE DESCRIPTION: SEQ ID NO: 3:

GGGGCGGCCG AGGTCTCGCA AGATCTGGA                                    29

(2) INFORMATION FOR SEQ ID NO: 4:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: Part non-coding strand lipase

(xi) SEQUENCE DESCRIPTION: SEQ ID NO: 4:

TTTGTCCAGG TCTTGCGAGA CCTCTCGACG AAT 33

(2) INFORMATION FOR SEQ ID NO: 5:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: Part coding strand lipase

(xi) SEQUENCE DESCRIPTION: SEQ ID NO: 5:

TTCGGGTTAA TTGGGACATG TCTTTAGTGC GA 32

(2) INFORMATION FOR SEQ ID NO: 6:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: primer lipo2

(xi) SEQUENCE DESCRIPTION: SEQ ID NO: 6:

CCCCAAGCTT AAGGCTAGCA AGACATGTCC CAATTAACCC 40

(2) INFORMATION FOR SEQ ID NO: 7:

(i) SEQUENCE CHARACTERISTICS:
(A) LENGTH: 894 base pairs
(B) TYPE: nucleic acid
(C) STRANDEDNESS: double
(D) TOPOLOGY: linear

(ii) MOLECULE TYPE: DNA (genomic)

(vi) ORIGINAL SOURCE:
(A) ORGANISM: Humicola lanuginosa

(ix) FEATURE:
(A) NAME/KEY: CDS
(B) LOCATION: 72..884
(D) OTHER INFORMATION: /product= "lipase"

(ix) FEATURE:
(A) NAME/KEY: mat_peptide
(B) LOCATION: 72..881
(D) OTHER INFORMATION: /product= "lipase"

(xi) SEQUENCE DESCRIPTION: SEQ ID NO: 7:

GAATTCGTAG CGACGATATG AGGAGCTCCC TTGTGCTGTT CTTTGTCTCT GCGTGGACGG 60

CCTTGGCCAC G GCC GAG GTC TCG CAA GAT CTG TTT AAC CAG TTC AAT CTC 110
Ala Glu Val Ser Gln Asp Leu Phe Asn Gln Phe Asn Leu
1               5                   10

TTT GCA CAG TAT TCT GCT GCC GCA TAC TGC GGA AAA AAC AAT GAT GCC 158
Phe Ala Gln Tyr Ser Ala Ala Ala Tyr Cys Gly Lys Asn Asn Asp Ala
    15                  20                  25

CCA GCT GGT ACA AAC ATT ACG TGC ACG GGA AAT GCC TGC CCC GAG GTA 206
Pro Ala Gly Thr Asn Ile Thr Cys Thr Gly Asn Ala Cys Pro Glu Val
30                  35                  40                  45

GAG AAG GCG GAT GCA ACG TTT CTC TAC TCG TTT GAA GAC TCT GGA GTG 254
Glu Lys Ala Asp Ala Thr Phe Leu Tyr Ser Phe Glu Asp Ser Gly Val
                50                  55                  60

GGC GAT GTC ACC GGC TTC CTT GCT CTA GAC AAC ACG AAC AAA TTG ATC 302
Gly Asp Val Thr Gly Phe Leu Ala Leu Asp Asn Thr Asn Lys Leu Ile
            65                  70                  75

GTC CTC TCT TTC CGT GGC TCT CGT TCC ATA GAA AAC TGG ATC GGA AAT 350
Val Leu Ser Phe Arg Gly Ser Arg Ser Ile Glu Asn Trp Ile Gly Asn
        80                  85                  90

CTT AAC TTC GAC TTG AAA GAA ATA AAT GAC ATT TGC TCC GGC TGC AGG 398
Leu Asn Phe Asp Leu Lys Glu Ile Asn Asp Ile Cys Ser Gly Cys Arg
    95                  100                 105

GGA CAT GAC GGC TTC ACC TCG AGC TGG AGG TCT GTA GCC GAT ACG TTA 446
Gly His Asp Gly Phe Thr Ser Ser Trp Arg Ser Val Ala Asp Thr Leu
110                 115                 120                 125

AGG CAG AAG GTG GAG GAT GCT GTG AGG GAG CAT CCC GAC TAT CGC GTG 494
Arg Gln Lys Val Glu Asp Ala Val Arg Glu His Pro Asp Tyr Arg Val
                130                 135                 140

GTG TTT ACC GGA CAT AGC TTG GGT GGT GCA TTG GCA ACT GTT GCC GGA 542
Val Phe Thr Gly His Ser Leu Gly Gly Ala Leu Ala Thr Val Ala Gly
            145                 150                 155

GCA GAC CTG CGT GGA AAT GGG TAT GAC ATC GAC GTG TTT TCA TAT GGC 590
Ala Asp Leu Arg Gly Asn Gly Tyr Asp Ile Asp Val Phe Ser Tyr Gly
        160                 165                 170

GCC CCC CGA GTC GGA AAC AGG GCT TTT GCA GAA TTC CTG ACC GTA CAG 638
Ala Pro Arg Val Gly Asn Arg Ala Phe Ala Glu Phe Leu Thr Val Gln
    175                 180                 185

ACC GGC GGT ACC CTC TAC CGC ATT ACC CAC ACC AAT GAT ATT GTC CCT 686
Thr Gly Gly Thr Leu Tyr Arg Ile Thr His Thr Asn Asp Ile Val Pro
190                 195                 200                 205

AGA CTC CCG CCG CGC GAG TTC GGT TAC AGC CAT TCT AGC CCA GAG TAC 734
Arg Leu Pro Pro Arg Glu Phe Gly Tyr Ser His Ser Ser Pro Glu Tyr
                210                 215                 220

TGG ATC AAA TCT GGA ACC CTT GTC CCC GTC ACC CGA AAC GAC ATC GTG 782
Trp Ile Lys Ser Gly Thr Leu Val Pro Val Thr Arg Asn Asp Ile Val
            225                 230                 235

AAG ATA GAA GGC ATC GAT GCC ACC GGC GGC AAT AAC CAG CCT AAC ATT 830
Lys Ile Glu Gly Ile Asp Ala Thr Gly Gly Asn Asn Gln Pro Asn Ile
        240                 245                 250

CCG GAT ATC CCT GCG CAC CTA TGG TAC TTC GGG TTA ATT GGG ACA TGT 878
Pro Asp Ile Pro Ala His Leu Trp Tyr Phe Gly Leu Ile Gly Thr Cys
    255                 260                 265

CTT TAGTGCGAAG CTT 894
Leu
270

(2) INFORMATION FOR SEQ ID NO: 8:

(i) SEQUENCE CHARACTERISTICS:
(A) LENGTH: 270 amino acids
(B) TYPE: amino acid
(D) TOPOLOGY: linear

(ii) MOLECULE TYPE: protein

(xi) SEQUENCE DESCRIPTION: SEQ ID NO: 8:

Ala Glu Val Ser Gln Asp Leu Phe Asn Gln Phe Asn Leu Phe Ala Gln
1               5                   10                  15

Tyr Ser Ala Ala Ala Tyr Cys Gly Lys Asn Asn Asp Ala Pro Ala Gly
            20                  25                  30

Thr Asn Ile Thr Cys Thr Gly Asn Ala Cys Pro Glu Val Glu Lys Ala
        35                  40                  45

Asp Ala Thr Phe Leu Tyr Ser Phe Glu Asp Ser Gly Val Gly Asp Val
    50                  55                  60

Thr Gly Phe Leu Ala Leu Asp Asn Thr Asn Lys Leu Ile Val Leu Ser
65                  70                  75                  80

Phe Arg Gly Ser Arg Ser Ile Glu Asn Trp Ile Gly Asn Leu Asn Phe
                85                  90                  95

Asp Leu Lys Glu Ile Asn Asp Ile Cys Ser Gly Cys Arg Gly His Asp
            100                 105                 110

Gly Phe Thr Ser Ser Trp Arg Ser Val Ala Asp Thr Leu Arg Gln Lys
        115                 120                 125

Val Glu Asp Ala Val Arg Glu His Pro Asp Tyr Arg Val Val Phe Thr
    130                 135                 140

Gly His Ser Leu Gly Gly Ala Leu Ala Thr Val Ala Gly Ala Asp Leu
145                 150                 155                 160

Arg Gly Asn Gly Tyr Asp Ile Asp Val Phe Ser Tyr Gly Ala Pro Arg
                165                 170                 175

Val Gly Asn Arg Ala Phe Ala Glu Phe Leu Thr Val Gln Thr Gly Gly
            180                 185                 190

Thr Leu Tyr Arg Ile Thr His Thr Asn Asp Ile Val Pro Arg Leu Pro
        195                 200                 205

Pro Arg Glu Phe Gly Tyr Ser His Ser Ser Pro Glu Tyr Trp Ile Lys
    210                 215                 220

Ser Gly Thr Leu Val Pro Val Thr Arg Asn Asp Ile Val Lys Ile Glu
225                 230                 235                 240

Gly Ile Asp Ala Thr Gly Gly Asn Asn Gln Pro Asn Ile Pro Asp Ile
                245                 250                 255

Pro Ala His Leu Trp Tyr Phe Gly Leu Ile Gly Thr Cys Leu
            260                 265                 270

(2) INFORMATION FOR SEQ ID NO: 9:

(i) SEQUENCE CHARACTERISTICS:
(A) LENGTH: 55 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: primer

(xi) SEQUENCE DESCRIPTION: SEQ ID NO: 9:

ATCCCTGCGC ACCTATGGTA CTTCGGGTTA ATTGGGACAT GTCTTGCTAG CCTTA 55

(2) INFORMATION FOR SEQ ID NO: 10:

(i) SEQUENCE CHARACTERISTICS:
(A) LENGTH: 59 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: primer

(xi) SEQUENCE DESCRIPTION: SEQ ID NO: 10:

AGCTTAAGGC TAGCAAGACA TGTCCCAATT AACCCGAAGT ACCATAGGTG CGCAGGGAT 59

(2) INFORMATION FOR SEQ ID NO: 11:

(i) SEQUENCE CHARACTERISTICS:
(A) LENGTH: 1828 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(A) ORGANISM: Geotrichum candidum
(B) STRAIN: CMICC 335426

(ix) FEATURE:
(A) NAME/KEY: CDS
(B) LOCATION: 40..1731
(D) OTHER INFORMATION: /product= "lipase"

(ix) FEATURE:
(A) NAME/KEY: sig_peptide
(B) LOCATION: 40..96

(ix) FEATURE:
(A) NAME/KEY: mat_peptide
(B) LOCATION: 97..1728
(D) OTHER INFORMATION: /product= "lipase"
/gene= "lipB"

(xi) SEQUENCE DESCRIPTION: SEQ ID NO: 11:

AATTCGGCAC GAGATTCCTT TGATTTGCAA CTGTTAATC ATG GTT TCC AAA AGC 54
Met Val Ser Lys Ser
-19 -15

TTT TTT TTG GCT GCG GCG CTC AAC GTA GTG GGC ACC TTG GCC CAG GCC 102
Phe Phe Leu Ala Ala Ala Leu Asn Val Val Gly Thr Leu Ala Gln Ala
-10 -5 1

CCC ACG GCC GTT CTT AAT GGC AAC GAG GTC ATC TCT GGT GTC CTT GAG 150
Pro Thr Ala Val Leu Asn Gly Asn Glu Val Ile Ser Gly Val Leu Glu
                5                   10                  15

GGC AAG GTT GAT ACC TTC AAG GGA ATC CCA TTT GCT GAC CCT CCT GTT 198
Gly Lys Val Asp Thr Phe Lys Gly Ile Pro Phe Ala Asp Pro Pro Val
            20                  25                  30

GGT GAC TTG CGG TTC AAG CAC CCC CAG CCT TTC ACT GGA TCC TAC CAG 246
Gly Asp Leu Arg Phe Lys His Pro Gln Pro Phe Thr Gly Ser Tyr Gln
35 40 45 50

GGT CTT AAG GCC AAC GAC TTC AGC TCT GCT TGT ATG CAG CTT GAT CCT 294
Gly Leu Lys Ala Asn Asp Phe Ser Ser Ala Cys Met Gln Leu Asp Pro
55 60 65

GGC AAT GCC TTT TCT TTG CTT GAC AAA GTA GTG GGC TTG GGA AAG ATT 342
Gly Asn Ala Phe Ser Leu Leu Asp Lys Val Val Gly Leu Gly Lys Ile
70 75 80

CTT CCT GAT AAC CTT AGA GGC CCT CTT TAT GAC ATG GCC CAG GGT AGT 390
Leu Pro Asp Asn Leu Arg Gly Pro Leu Tyr Asp Met Ala Gln Gly Ser
                85                  90                  95

GTC TCC ATG AAT GAG GAC TGT CTC TAC CTT AAC GTT TTC CGC CCC GCT 438
Val Ser Met Asn Glu Asp Cys Leu Tyr Leu Asn Val Phe Arg Pro Ala
            100                 105                 110

GGC ACC AAG CCT GAT GCT AAG CTC CCC GTC ATG GTT TGG ATT TAC GGT 486
Gly Thr Lys Pro Asp Ala Lys Leu Pro Val Met Val Trp Ile Tyr Gly
115 120 125 130

GGT GCC TTT GTG TTT GGT TCT TCT GCT TCT TAC CCT GGT AAC GGC TAC 534
Gly Ala Phe Val Phe Gly Ser Ser Ala Ser Tyr Pro Gly Asn Gly Tyr
135 140 145

GTC AAG GAG AGT GTG GAA ATG GGC CAG CCT GTT GTG TTT GTT TCC ATC 582
Val Lys Glu Ser Val Glu Met Gly Gln Pro Val Val Phe Val Ser Ile
150 155 160

AAC TAC CGT ACC GGC CCC TAT GGA TTC TTG GGT GGT GAT GCC ATC ACC 630
Asn Tyr Arg Thr Gly Pro Tyr Gly Phe Leu Gly Gly Asp Ala Ile Thr
                165                 170                 175

GCT GAG GGC AAC ACC AAC GCT GGT CTG CAC GAC CAG CGC AAG GGT CTC 678
Ala Glu Gly Asn Thr Asn Ala Gly Leu His Asp Gln Arg Lys Gly Leu
            180                 185                 190

GAG TGG GTT AGC GAC AAC ATT GCC AAC TTT GGT GGT GAT CCC GAC AAG 726
Glu Trp Val Ser Asp Asn Ile Ala Asn Phe Gly Gly Asp Pro Asp Lys
195 200 205 210

GTC ATG ATT TTC GGT GAG TCC GCT GGT GCC ATG AGT GTT GCT CAC CAG 774
Val Met Ile Phe Gly Glu Ser Ala Gly Ala Met Ser Val Ala His Gln
215 220 225

CTT GTT GCC TAC GGT GGT GAC AAC ACC TAC AAC GGA AAG CAG CTT TTC 822
Leu Val Ala Tyr Gly Gly Asp Asn Thr Tyr Asn Gly Lys Gln Leu Phe
230 235 240

CAC TCT GCC ATT CTT CAG TCT GGC GGT CCT CTT CCT TAC TTT GAC TCT 870
His Ser Ala Ile Leu Gln Ser Gly Gly Pro Leu Pro Tyr Phe Asp Ser
                245                 250                 255

ACT TCT GTT GGT CCC GAG AGT GCC TAC AGC AGA TTT GCT CAG TAT GCC 918
Thr Ser Val Gly Pro Glu Ser Ala Tyr Ser Arg Phe Ala Gln Tyr Ala
            260                 265                 270

GGA TGT GAC ACC AGT GCC AGT GAT AAT GAC ACT CTG GCT TGT CTC CGC 966
Gly Cys Asp Thr Ser Ala Ser Asp Asn Asp Thr Leu Ala Cys Leu Arg
275 280 285 290

AGC AAG TCC AGC GAT GTC TTG CAC AGT GCG CAG AAC TCG TAT GAT CTT 1014
Ser Lys Ser Ser Asp Val Leu His Ser Ala Gln Asn Ser Tyr Asp Leu
295 300 305

AAG GAC CTG TTT GGT CTG CTC CCT CAA TTC CTT GGA TTT GGT CCC AGA 1062
Lys Asp Leu Phe Gly Leu Leu Pro Gln Phe Leu Gly Phe Gly Pro Arg
310 315 320

CCC GAC GGC AAC ATT ATT CCC GAT GCC GCT TAT GAG CTC TAC CGC AGC 1110
Pro Asp Gly Asn Ile Ile Pro Asp Ala Ala Tyr Glu Leu Tyr Arg Ser
                325                 330                 335

GGT AGA TAC GCC AAG GTT CCC TAC ATT ACT GGC AAC CAG GAG GAT GAG 1158
Gly Arg Tyr Ala Lys Val Pro Tyr Ile Thr Gly Asn Gln Glu Asp Glu
            340                 345                 350

GGT ACT ATT CTT GCC CCC GTT GCT ATT AAT GCT ACC ACT ACT CCC CAT 1206
Gly Thr Ile Leu Ala Pro Val Ala Ile Asn Ala Thr Thr Thr Pro His
355 360 365 370

GTT AAG AAG TGG TTG AAG TAC ATT TGT AGC CAG GCT TCT GAC GCT TCG 1254
Val Lys Lys Trp Leu Lys Tyr Ile Cys Ser Gln Ala Ser Asp Ala Ser
375 380 385

CTT GAT CGT GTT TTG TCG CTC TAC CCC GGC TCT TGG TCG GAG GGT TCA 1302
Leu Asp Arg Val Leu Ser Leu Tyr Pro Gly Ser Trp Ser Glu Gly Ser
390 395 400

CCA TTC CGC ACT GGT ATT CTT AAT GCT CTT ACC CCT CAG TTC AAG CGC 1350
Pro Phe Arg Thr Gly Ile Leu Asn Ala Leu Thr Pro Gln Phe Lys Arg
                405                 410                 415

ATT GCT GCC ATT TTC ACT GAT TTG CTG TTC CAG TCT CCT CGT CGT GTT 1398
Ile Ala Ala Ile Phe Thr Asp Leu Leu Phe Gln Ser Pro Arg Arg Val
            420                 425                 430

ATG CTT AAC GCT ACC AAG GAC GTC AAC CGC TGG ACT TAC CTT GCC ACC 1446
Met Leu Asn Ala Thr Lys Asp Val Asn Arg Trp Thr Tyr Leu Ala Thr
435 440 445 450

CAG CTC CAT AAC CTC GTT CCA TTT TTG GGT ACT TTC CAT GGC AGT GAT 1494
Gln Leu His Asn Leu Val Pro Phe Leu Gly Thr Phe His Gly Ser Asp
455 460 465

CTT CTT TTT CAA TAC TAC GTG GAC CTT GGC CCA TCT TCT GCT TAC CGC 1542
Leu Leu Phe Gln Tyr Tyr Val Asp Leu Gly Pro Ser Ser Ala Tyr Arg
470 475 480

CGC TAC TTT ATC TCG TTT GCC AAC CAC CAC GAC CCC AAC GTT GGT ACC 1590
Arg Tyr Phe Ile Ser Phe Ala Asn His His Asp Pro Asn Val Gly Thr
                485                 490                 495

AAC CTC CAA CAG TGG GAT ATG TAC ACT GAT GCA GGC AAG GAG ATG CTT 1638
Asn Leu Gln Gln Trp Asp Met Tyr Thr Asp Ala Gly Lys Glu Met Leu
            500                 505                 510

CAG ATT CAT ATG ATT GGT AAC TCT ATG AGA ACT GAC GAC TTT AGA ATC 1686
Gln Ile His Met Ile Gly Asn Ser Met Arg Thr Asp Asp Phe Arg Ile
515 520 525 530

GAG GGA ATC TCG AAC TTT GAG TCT GAC GTT ACT CTC TTC GGT TAATCCCATT 1738
Glu Gly Ile Ser Asn Phe Glu Ser Asp Val Thr Leu Phe Gly
535 540 545

TAGCAAGTTT TGTGTATTTC AAGTATACCA GTTGATGTAA TATATCAATA GATTACAAAT 1798

TAATTAGTGA AAAAAAAAAA AAAAAAAAAC 1828

(2) INFORMATION FOR SEQ ID NO: 12:

(i) SEQUENCE CHARACTERISTICS:
(A) LENGTH: 563 amino acids
(B) TYPE: amino acid
(D) TOPOLOGY: linear

(ii) MOLECULE TYPE: protein

(xi) SEQUENCE DESCRIPTION: SEQ ID NO: 12:

Met Val Ser Lys Ser Phe Phe Leu Ala Ala Ala Leu Asn Val Val Gly
-19             -15                 -10                 -5

Thr Leu Ala Gln Ala Pro Thr Ala Val Leu Asn Gly Asn Glu Val Ile
            1               5                   10

Ser Gly Val Leu Glu Gly Lys Val Asp Thr Phe Lys Gly Ile Pro Phe
    15                  20                  25

Ala Asp Pro Pro Val Gly Asp Leu Arg Phe Lys His Pro Gln Pro Phe
30                  35                  40                  45

Thr Gly Ser Tyr Gln Gly Leu Lys Ala Asn Asp Phe Ser Ser Ala Cys
                50                  55                  60

Met Gln Leu Asp Pro Gly Asn Ala Phe Ser Leu Leu Asp Lys Val Val
            65                  70                  75

Gly Leu Gly Lys Ile Leu Pro Asp Asn Leu Arg Gly Pro Leu Tyr Asp
        80                  85                  90

Met Ala Gln Gly Ser Val Ser Met Asn Glu Asp Cys Leu Tyr Leu Asn
    95                  100                 105

Val Phe Arg Pro Ala Gly Thr Lys Pro Asp Ala Lys Leu Pro Val Met
110                 115                 120                 125

Val Trp Ile Tyr Gly Gly Ala Phe Val Phe Gly Ser Ser Ala Ser Tyr
                130                 135                 140

Pro Gly Asn Gly Tyr Val Lys Glu Ser Val Glu Met Gly Gln Pro Val
            145                 150                 155

Val Phe Val Ser Ile Asn Tyr Arg Thr Gly Pro Tyr Gly Phe Leu Gly
        160                 165                 170

Gly Asp Ala Ile Thr Ala Glu Gly Asn Thr Asn Ala Gly Leu His Asp
    175                 180                 185

Gln Arg Lys Gly Leu Glu Trp Val Ser Asp Asn Ile Ala Asn Phe Gly
190                 195                 200                 205

Gly Asp Pro Asp Lys Val Met Ile Phe Gly Glu Ser Ala Gly Ala Met
    210                 215                 220

Ser Val Ala His Gln Leu Val Ala Tyr Gly Gly Asp Asn Thr Tyr Asn
225 230 235

Gly Lys Gln Leu Phe His Ser Ala Ile Leu Gln Ser Gly Gly Pro Leu
240 245 250

Pro Tyr Phe Asp Ser Thr Ser Val Gly Pro Glu Ser Ala Tyr Ser Arg
255 260 265

Phe Ala Gln Tyr Ala Gly Cys Asp Thr Ser Ala Ser Asp Asn Asp Thr
270 275 280 285

Leu Ala Cys Leu Arg Ser Lys Ser Ser Asp Val Leu His Ser Ala Gln
    290                 295                 300

Asn Ser Tyr Asp Leu Lys Asp Leu Phe Gly Leu Leu Pro Gln Phe Leu
305 310 315

Gly Phe Gly Pro Arg Pro Asp Gly Asn Ile Ile Pro Asp Ala Ala Tyr
320 325 330

Glu Leu Tyr Arg Ser Gly Arg Tyr Ala Lys Val Pro Tyr Ile Thr Gly
335 340 345

Asn Gln Glu Asp Glu Gly Thr Ile Leu Ala Pro Val Ala Ile Asn Ala
350 355 360 365

Thr Thr Thr Pro His Val Lys Lys Trp Leu Lys Tyr Ile Cys Ser Gln
    370                 375                 380

Ala Ser Asp Ala Ser Leu Asp Arg Val Leu Ser Leu Tyr Pro Gly Ser
385 390 395

Trp Ser Glu Gly Ser Pro Phe Arg Thr Gly Ile Leu Asn Ala Leu Thr
400 405 410

Pro Gln Phe Lys Arg Ile Ala Ala Ile Phe Thr Asp Leu Leu Phe Gln
415 420 425

Ser Pro Arg Arg Val Met Leu Asn Ala Thr Lys Asp Val Asn Arg Trp
430 435 440 445

Thr Tyr Leu Ala Thr Gln Leu His Asn Leu Val Pro Phe Leu Gly Thr
    450                 455                 460

Phe His Gly Ser Asp Leu Leu Phe Gln Tyr Tyr Val Asp Leu Gly Pro
465 470 475

Ser Ser Ala Tyr Arg Arg Tyr Phe Ile Ser Phe Ala Asn His His Asp
480 485 490

Pro Asn Val Gly Thr Asn Leu Gln Gln Trp Asp Met Tyr Thr Asp Ala
495 500 505

Gly Lys Glu Met Leu Gln Ile His Met Ile Gly Asn Ser Met Arg Thr
510 515 520 525

Asp Asp Phe Arg Ile Glu Gly Ile Ser Asn Phe Glu Ser Asp Val Thr
    530                 535                 540

Leu Phe Gly

(2) INFORMATION FOR SEQ ID NO: 13:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: primer lipo3

(xi) SEQUENCE DESCRIPTION: SEQ ID NO: 13:

GGGGCGGCCG CGCAGGCCCC AAGGCGGTCT CTCAAT

(2) INFORMATION FOR SEQ ID NO: 14:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: Part non-coding strand lipaseII

(xi) SEQUENCE DESCRIPTION: SEQ ID NO: 14:

ATTGAGAGAC CGCCGTGGGG CCTGGGCCAG 30

(2) INFORMATION FOR SEQ ID NO: 15:

(i) SEQUENCE CHARACTERISTICS:
(A) LENGTH: 38 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: Part coding strand lipaseII

(xi) SEQUENCE DESCRIPTION: SEQ ID NO: 15:

CAAACTTTGA GACTGACGTT AATCTCTACG GTTAAAAC 38

(2) INFORMATION FOR SEQ ID NO: 16:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: primer lipo4

(xi) SEQUENCE DESCRIPTION: SEQ ID NO: 16:

CCCCGCTAGC ACCGTAGAGA TTAACGTCAG TC 32

(2) INFORMATION FOR SEQ ID NO: 17:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: primer lipo5

(xi) SEQUENCE DESCRIPTION: SEQ ID NO: 17:

CCCGCGGCCG CGAGCATTGA TGGTGGTATC 30

(2) INFORMATION FOR SEQ ID NO: 18:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: Part non-coding strand lipase

(xi) SEQUENCE DESCRIPTION: SEQ ID NO: 18:

GATACCACGA TCAATGCT 18

(2) INFORMATION FOR SEQ ID NO: 19:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: Part coding strand lipase

(xi) SEQUENCE DESCRIPTION: SEQ ID NO: 19:

AACACAGGCC TCTGTACT 18

(2) INFORMATION FOR SEQ ID NO: 20:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: primer lipo6

(xi) SEQUENCE DESCRIPTION: SEQ ID NO: 20:

CCGCGCTAGC AGTACAGAGG CCTGTGTT 28

(2) INFORMATION FOR SEQ ID NO: 21:

(i) SEQUENCE CHARACTERISTICS:
(A) LENGTH: 2685 base pairs
(B) TYPE: nucleic acid
(C) STRANDEDNESS: double
(D) TOPOLOGY: linear

(ii) MOLECULE TYPE: DNA (genomic)

(vi) ORIGINAL SOURCE:
(A) ORGANISM: Saccharomyces cerevisiae

(vii) IMMEDIATE SOURCE:
(B) CLONE: pYY105

(ix) FEATURE:
(A) NAME/KEY: CDS
(B) LOCATION: 1..2685
(D) OTHER INFORMATION: /product= "Flocculation protein"
/gene= "FLO1"

(xi) SEQUENCE DESCRIPTION: SEQ ID NO: 21:

ATG ACA ATG CCT CAT CGC TAT ATG TTT TTG GCA GTC TTT ACA CTT CTG 48
Met Thr Met Pro His Arg Tyr Met Phe Leu Ala Val Phe Thr Leu Leu
1               5                   10                  15

GCA CTA ACT AGT GTG GCC TCA GGA GCC ACA GAG GCG TGC TTA CCA GCA 96
Ala Leu Thr Ser Val Ala Ser Gly Ala Thr Glu Ala Cys Leu Pro Ala
            20                  25                  30

GGC CAG AGG AAA AGT GGG ATG AAT ATA AAT TTT TAC CAG TAT TCA TTG 144
Gly Gln Arg Lys Ser Gly Met Asn Ile Asn Phe Tyr Gln Tyr Ser Leu
        35                  40                  45

AAA GAT TCC TCC ACA TAT TCG AAT GCA GCA TAT ATG GCT TAT GGA TAT 192
Lys Asp Ser Ser Thr Tyr Ser Asn Ala Ala Tyr Met Ala Tyr Gly Tyr
    50                  55                  60

GCC TCA AAA ACC AAA CTA GGT TCT GTC GGA GGA CAA ACT GAT ATC TCG 240
Ala Ser Lys Thr Lys Leu Gly Ser Val Gly Gly Gln Thr Asp Ile Ser
65                  70                  75                  80

ATT GAT TAT AAT ATT CCC TGT GTT AGT TCA TCA GGC ACA TTT CCT TGT 288
Ile Asp Tyr Asn Ile Pro Cys Val Ser Ser Ser Gly Thr Phe Pro Cys
                85                  90                  95

CCT CAA GAA GAT TCC TAT GGA AAC TGG GGA TGC AAA GGA ATG GGT GCT 336
Pro Gln Glu Asp Ser Tyr Gly Asn Trp Gly Cys Lys Gly Met Gly Ala
            100                 105                 110

TGT TCT AAT AGT CAA GGA ATT GCA TAC TGG AGT ACT GAT TTA TTT GGT 384
Cys Ser Asn Ser Gln Gly Ile Ala Tyr Trp Ser Thr Asp Leu Phe Gly
        115                 120                 125

TTC TAT ACT ACC CCA ACA AAC GTA ACC CTA GAA ATG ACA GGT TAT TTT 432
Phe Tyr Thr Thr Pro Thr Asn Val Thr Leu Glu Met Thr Gly Tyr Phe
    130                 135                 140

TTA CCA CCA CAG ACG GGT TCT TAC ACA TTC AAG TTT GCT ACA GTT GAC 480
Leu Pro Pro Gln Thr Gly Ser Tyr Thr Phe Lys Phe Ala Thr Val Asp
145                 150                 155                 160

GAC TCT GCA ATT CTA TCA GTA GGT GGT GCA ACC GCG TTC AAC TGT TGT 528
Asp Ser Ala Ile Leu Ser Val Gly Gly Ala Thr Ala Phe Asn Cys Cys
                165                 170                 175

GCT CAA CAG CAA CCG CCG ATC ACA TCA ACG AAC TTT ACC ATT GAC GGT 576
Ala Gln Gln Gln Pro Pro Ile Thr Ser Thr Asn Phe Thr Ile Asp Gly
            180                 185                 190

ATC AAG CCA TGG GGT GGA AGT TTG CCA CCT AAT ATC GAA GGA ACC GTC 624
Ile Lys Pro Trp Gly Gly Ser Leu Pro Pro Asn Ile Glu Gly Thr Val
        195                 200                 205

TAT ATG TAC GCT GGC TAC TAT TAT CCA ATG AAG GTT GTT TAC TCG AAC 672
Tyr Met Tyr Ala Gly Tyr Tyr Tyr Pro Met Lys Val Val Tyr Ser Asn
    210                 215                 220

GCT GTT TCT TGG GGT ACA CTT CCA ATT AGT GTG ACA CTT CCA GAT GGT 720
Ala Val Ser Trp Gly Thr Leu Pro Ile Ser Val Thr Leu Pro Asp Gly
225                 230                 235                 240

ACC ACT GTA AGT GAT GAC TTC GAA GGG TAC GTC TAT TCC TTT GAC GAT 768
Thr Thr Val Ser Asp Asp Phe Glu Gly Tyr Val Tyr Ser Phe Asp Asp
                245                 250                 255

GAC CTA AGT CAA TCT AAC TGT ACT GTC CCT GAC CCT TCA AAT TAT GCT 816
Asp Leu Ser Gln Ser Asn Cys Thr Val Pro Asp Pro Ser Asn Tyr Ala
            260                 265                 270

GTC AGT ACC ACT ACA ACT ACA ACG GAA CCA TGG ACC GGT ACT TTC ACT 864
Val Ser Thr Thr Thr Thr Thr Thr Glu Pro Trp Thr Gly Thr Phe Thr
        275                 280                 285

TCT ACA TCT ACT GAA ATG ACC ACC GTC ACC GGT ACC AAC GGC GTT CCA 912
Ser Thr Ser Thr Glu Met Thr Thr Val Thr Gly Thr Asn Gly Val Pro
    290                 295                 300

ACT GAC GAA ACC GTC ATT GTC ATC AGA ACT CCA ACC AGT GAA GGT CTA 960
Thr Asp Glu Thr Val Ile Val Ile Arg Thr Pro Thr Ser Glu Gly Leu
305                 310                 315                 320

ATC AGC ACC ACC ACT GAA CCA TGG ACT GGC ACT TTC ACT TCG ACT TCC 1008
Ile Ser Thr Thr Thr Glu Pro Trp Thr Gly Thr Phe Thr Ser Thr Ser
                325                 330                 335

ACT GAG GTT ACC ACC ATC ACT GGA ACC AAC GGT CAA CCA ACT GAC GAA 1056
Thr Glu Val Thr Thr Ile Thr Gly Thr Asn Gly Gln Pro Thr Asp Glu
            340                 345                 350

ACT GTG ATT GTT ATC AGA ACT CCA ACC AGT GAA GGT CTA ATC AGC ACC 1104
Thr Val Ile Val Ile Arg Thr Pro Thr Ser Glu Gly Leu Ile Ser Thr
        355                 360                 365

ACC ACT GAA CCA TGG ACT GGT ACT TTC ACT TCT ACA TCT ACT GAA ATG 1152
Thr Thr Glu Pro Trp Thr Gly Thr Phe Thr Ser Thr Ser Thr Glu Met
    370                 375                 380

ACC ACC GTC ACC GGT ACT AAC GGT CAA CCA ACT GAC GAA ACC GTG ATT 1200
Thr Thr Val Thr Gly Thr Asn Gly Gln Pro Thr Asp Glu Thr Val Ile
385                 390                 395                 400

GTT ATC AGA ACT CCA ACC AGT GAA GGT TTG GTT ACA ACC ACC ACT GAA 1248
Val Ile Arg Thr Pro Thr Ser Glu Gly Leu Val Thr Thr Thr Thr Glu
                405                 410                 415

CCA TGG ACT GGT ACT TTT ACT TCG ACT TCC ACT GAA ATG TCT ACT GTC 1296
Pro Trp Thr Gly Thr Phe Thr Ser Thr Ser Thr Glu Met Ser Thr Val
            420                 425                 430

ACT GGA ACC AAT GGC TTG CCA ACT GAT GAA ACT GTC ATT GTT GTC AAA 1344
Thr Gly Thr Asn Gly Leu Pro Thr Asp Glu Thr Val Ile Val Val Lys
        435                 440                 445

ACT CCA ACT ACT GCC ATC TCA TCC AGT TTG TCA TCA TCA TCT TCA GGA 1392
Thr Pro Thr Thr Ala Ile Ser Ser Ser Leu Ser Ser Ser Ser Ser Gly
    450                 455                 460

CAA ATC ACC AGC TCT ATC ACG TCT TCG CGT CCA ATT ATT ACC CCA TTC 1440
Gln Ile Thr Ser Ser Ile Thr Ser Ser Arg Pro Ile Ile Thr Pro Phe
465                 470                 475                 480

TAT CCT AGC AAT GGA ACT TCT GTG ATT TCT TCC TCA GTA ATT TCT TCC 1488
Tyr Pro Ser Asn Gly Thr Ser Val Ile Ser Ser Ser Val Ile Ser Ser
                485                 490                 495

TCA GTC ACT TCT TCT CTA TTC ACT TCT TCT CCA GTC ATT TCT TCC TCA 1536
Ser Val Thr Ser Ser Leu Phe Thr Ser Ser Pro Val Ile Ser Ser Ser
            500                 505                 510

GTC ATT TCT TCT TCT ACA ACA ACC TCC ACT TCT ATA TTT TCT GAA TCA 1584
Val Ile Ser Ser Ser Thr Thr Thr Ser Thr Ser Ile Phe Ser Glu Ser
        515                 520                 525

TCT AAA TCA TCC GTC ATT CCA ACC AGT AGT TCC ACC TCT GGT TCT TCT 1632
Ser Lys Ser Ser Val Ile Pro Thr Ser Ser Ser Thr Ser Gly Ser Ser
    530                 535                 540

GAG AGC GAA ACG AGT TCA GCT GGT TCT GTC TCT TCT TCC TCT TTT ATC 1680
Glu Ser Glu Thr Ser Ser Ala Gly Ser Val Ser Ser Ser Ser Phe Ile
545                 550                 555                 560

TCT TCT GAA TCA TCA AAA TCT CCT ACA TAT TCT TCT TCA TCA TTA CCA 1728
Ser Ser Glu Ser Ser Lys Ser Pro Thr Tyr Ser Ser Ser Ser Leu Pro
                565                 570                 575

CTT GTT ACC AGT GCG ACA ACA AGC CAG GAA ACT GCT TCT TCA TTA CCA 1776
Leu Val Thr Ser Ala Thr Thr Ser Gln Glu Thr Ala Ser Ser Leu Pro
            580                 585                 590

CCT GCT ACC ACT ACA AAA ACG AGC GAA CAA ACC ACT TTG GTT ACC GTG 1824
Pro Ala Thr Thr Thr Lys Thr Ser Glu Gln Thr Thr Leu Val Thr Val
        595                 600                 605

ACA TCC TGC GAG TCT CAT GTG TGC ACT GAA TCC ATC TCC CCT GCG ATT 1872
Thr Ser Cys Glu Ser His Val Cys Thr Glu Ser Ile Ser Pro Ala Ile
    610                 615                 620

GTT TCC ACA GCT ACT GTT ACT GTT AGC GGC GTC ACA ACA GAG TAT ACC 1920
Val Ser Thr Ala Thr Val Thr Val Ser Gly Val Thr Thr Glu Tyr Thr
625                 630                 635                 640

ACA TGG TGC CCT ATT TCT ACT ACA GAG ACA ACA AAG CAA ACC AAA GGG 1968
Thr Trp Cys Pro Ile Ser Thr Thr Glu Thr Thr Lys Gln Thr Lys Gly
                645                 650                 655

ACA ACA GAG CAA ACC ACA GAA ACA ACA AAA CAA ACC ACG GTA GTT ACA 2016
Thr Thr Glu Gln Thr Thr Glu Thr Thr Lys Gln Thr Thr Val Val Thr
            660                 665                 670

ATT TCT TCT TGT GAA TCT GAC GTA TGC TCT AAG ACT GCT TCT CCA GCC 2064
Ile Ser Ser Cys Glu Ser Asp Val Cys Ser Lys Thr Ala Ser Pro Ala
        675                 680                 685

ATT GTA TCT ACA AGC ACT GCT ACT ATT AAC GGC GTT ACT ACA GAA TAC 2112
Ile Val Ser Thr Ser Thr Ala Thr Ile Asn Gly Val Thr Thr Glu Tyr
    690                 695                 700

ACA ACA TGG TGT CCT ATT TCC ACC ACA GAA TCG AGG CAA CAA ACA ACG 2160
Thr Thr Trp Cys Pro Ile Ser Thr Thr Glu Ser Arg Gln Gln Thr Thr
705                 710                 715                 720

CTA GTT ACT GTT ACT TCC TGC GAA TCT GGT GTG TGT TCC GAA ACT GCT 2208
Leu Val Thr Val Thr Ser Cys Glu Ser Gly Val Cys Ser Glu Thr Ala
                725                 730                 735

TCA CCT GCC ATT GTT TCG ACG GCC ACG GCT ACT GTG AAT GAT GTT GTT 2256
Ser Pro Ala Ile Val Ser Thr Ala Thr Ala Thr Val Asn Asp Val Val
            740                 745                 750

ACG GTC TAT CCT ACA TGG AGG CCA CAG ACT GCG AAT GAA GAG TCT GTC 2304
Thr Val Tyr Pro Thr Trp Arg Pro Gln Thr Ala Asn Glu Glu Ser Val
        755                 760                 765

AGC TCT AAA ATG AAC AGT GCT ACC GGT GAG ACA ACA ACC AAT ACT TTA 2352
Ser Ser Lys Met Asn Ser Ala Thr Gly Glu Thr Thr Thr Asn Thr Leu
    770                 775                 780

GCT GCT GAA ACG ACT ACC AAT ACT GTA GCT GCT GAG ACG ATT ACC AAT 2400
Ala Ala Glu Thr Thr Thr Asn Thr Val Ala Ala Glu Thr Ile Thr Asn
785                 790                 795                 800

ACT GGA GCT GCT GAG ACG AAA ACA GTA GTC ACC TCT TCG CTT TCA AGA 2448
Thr Gly Ala Ala Glu Thr Lys Thr Val Val Thr Ser Ser Leu Ser Arg
                805                 810                 815

TCT AAT CAC GCT GAA ACA CAG ACG GCT TCC GCG ACC GAT GTG ATT GGT 2496
Ser Asn His Ala Glu Thr Gln Thr Ala Ser Ala Thr Asp Val Ile Gly
            820                 825                 830

CAC AGC AGT AGT GTT GTT TCT GTA TCC GAA ACT GGC AAC ACC AAG AGT 2544
His Ser Ser Ser Val Val Ser Val Ser Glu Thr Gly Asn Thr Lys Ser
        835                 840                 845

CTA ACA AGT TCC GGG TTG AGT ACT ATG TCG CAA CAG CCT CGT AGC ACA 2592
Leu Thr Ser Ser Gly Leu Ser Thr Met Ser Gln Gln Pro Arg Ser Thr
    850                 855                 860

CCA GCA AGC AGC ATG GTA GGA TAT AGT ACA GCT TCT TTA GAA ATT TCA 2640
Pro Ala Ser Ser Met Val Gly Tyr Ser Thr Ala Ser Leu Glu Ile Ser
865                 870                 875                 880

ACG TAT GCT GGC AGT GCA ACA GCT TAC TGG CCG GTA GTG GTT TAA 2686
Thr Tyr Ala Gly Ser Ala Thr Ala Tyr Trp Pro Val Val Val
885 890 895

(2) INFORMATION FOR SEQ ID NO: 22:

(i) SEQUENCE CHARACTERISTICS:
(A) LENGTH: 894 amino acids
(B) TYPE: amino acid
(D) TOPOLOGY: linear

(ii) MOLECULE TYPE: protein

(xi) SEQUENCE DESCRIPTION: SEQ ID NO: 22:

Met Thr Met Pro His Arg Tyr Met Phe Leu Ala Val Phe Thr Leu Leu
1               5                   10                  15

Ala Leu Thr Ser Val Ala Ser Gly Ala Thr Glu Ala Cys Leu Pro Ala
            20                  25                  30

Gly Gln Arg Lys Ser Gly Met Asn Ile Asn Phe Tyr Gln Tyr Ser Leu
        35                  40                  45

Lys Asp Ser Ser Thr Tyr Ser Asn Ala Ala Tyr Met Ala Tyr Gly Tyr
    50                  55                  60

Ala Ser Lys Thr Lys Leu Gly Ser Val Gly Gly Gln Thr Asp Ile Ser
65                  70                  75                  80

Ile Asp Tyr Asn Ile Pro Cys Val Ser Ser Ser Gly Thr Phe Pro Cys
                85                  90                  95

Pro Gln Glu Asp Ser Tyr Gly Asn Trp Gly Cys Lys Gly Met Gly Ala
            100                 105                 110

Cys Ser Asn Ser Gln Gly Ile Ala Tyr Trp Ser Thr Asp Leu Phe Gly
        115                 120                 125

Phe Tyr Thr Thr Pro Thr Asn Val Thr Leu Glu Met Thr Gly Tyr Phe
    130                 135                 140

Leu Pro Pro Gln Thr Gly Ser Tyr Thr Phe Lys Phe Ala Thr Val Asp
145                 150                 155                 160

Asp Ser Ala Ile Leu Ser Val Gly Gly Ala Thr Ala Phe Asn Cys Cys
                165                 170                 175

Ala Gln Gln Gln Pro Pro Ile Thr Ser Thr Asn Phe Thr Ile Asp Gly
            180                 185                 190

Ile Lys Pro Trp Gly Gly Ser Leu Pro Pro Asn Ile Glu Gly Thr Val
        195                 200                 205

Tyr Met Tyr Ala Gly Tyr Tyr Tyr Pro Met Lys Val Val Tyr Ser Asn
    210                 215                 220

Ala Val Ser Trp Gly Thr Leu Pro Ile Ser Val Thr Leu Pro Asp Gly
225                 230                 235                 240

Thr Thr Val Ser Asp Asp Phe Glu Gly Tyr Val Tyr Ser Phe Asp Asp
                245                 250                 255

Asp Leu Ser Gln Ser Asn Cys Thr Val Pro Asp Pro Ser Asn Tyr Ala
            260                 265                 270

Val Ser Thr Thr Thr Thr Thr Thr Glu Pro Trp Thr Gly Thr Phe Thr
        275                 280                 285

Ser Thr Ser Thr Glu Met Thr Thr Val Thr Gly Thr Asn Gly Val Pro
    290                 295                 300

Thr Asp Glu Thr Val Ile Val Ile Arg Thr Pro Thr Ser Glu Gly Leu
305                 310                 315                 320

Ile Ser Thr Thr Thr Glu Pro Trp Thr Gly Thr Phe Thr Ser Thr Ser
                325                 330                 335

Thr Glu Val Thr Thr Ile Thr Gly Thr Asn Gly Gln Pro Thr Asp Glu
            340                 345                 350

Thr Val Ile Val Ile Arg Thr Pro Thr Ser Glu Gly Leu Ile Ser Thr
        355                 360                 365

Thr Thr Glu Pro Trp Thr Gly Thr Phe Thr Ser Thr Ser Thr Glu Met
    370                 375                 380

Thr Thr Val Thr Gly Thr Asn Gly Gln Pro Thr Asp Glu Thr Val Ile
385                 390                 395                 400

Val Ile Arg Thr Pro Thr Ser Glu Gly Leu Val Thr Thr Thr Thr Glu
                405                 410                 415

Pro Trp Thr Gly Thr Phe Thr Ser Thr Ser Thr Glu Met Ser Thr Val
            420                 425                 430

Thr Gly Thr Asn Gly Leu Pro Thr Asp Glu Thr Val Ile Val Val Lys
        435                 440                 445

Thr Pro Thr Thr Ala Ile Ser Ser Ser Leu Ser Ser Ser Ser Ser Gly
    450                 455                 460

Gln Ile Thr Ser Ser Ile Thr Ser Ser Arg Pro Ile Ile Thr Pro Phe
465                 470                 475                 480

Tyr Pro Ser Asn Gly Thr Ser Val Ile Ser Ser Ser Val Ile Ser Ser
                485                 490                 495

Ser Val Thr Ser Ser Leu Phe Thr Ser Ser Pro Val Ile Ser Ser Ser
            500                 505                 510

Val Ile Ser Ser Ser Thr Thr Thr Ser Thr Ser Ile Phe Ser Glu Ser
        515                 520                 525

Ser Lys Ser Ser Val Ile Pro Thr Ser Ser Ser Thr Ser Gly Ser Ser
    530                 535                 540

Glu Ser Glu Thr Ser Ser Ala Gly Ser Val Ser Ser Ser Ser Phe Ile
545                 550                 555                 560

Ser Ser Glu Ser Ser Lys Ser Pro Thr Tyr Ser Ser Ser Ser Leu Pro
                565                 570                 575

Leu Val Thr Ser Ala Thr Thr Ser Gln Glu Thr Ala Ser Ser Leu Pro
            580                 585                 590

Pro Ala Thr Thr Thr Lys Thr Ser Glu Gln Thr Thr Leu Val Thr Val
        595                 600                 605

Thr Ser Cys Glu Ser His Val Cys Thr Glu Ser Ile Ser Pro Ala Ile
    610                 615                 620

Val Ser Thr Ala Thr Val Thr Val Ser Gly Val Thr Thr Glu Tyr Thr
625                 630                 635                 640

Thr Trp Cys Pro Ile Ser Thr Thr Glu Thr Thr Lys Gln Thr Lys Gly
                645                 650                 655

Thr Thr Glu Gln Thr Thr Glu Thr Thr Lys Gln Thr Thr Val Val Thr
            660                 665                 670

Ile Ser Ser Cys Glu Ser Asp Val Cys Ser Lys Thr Ala Ser Pro Ala
        675                 680                 685

Ile Val Ser Thr Ser Thr Ala Thr Ile Asn Gly Val Thr Thr Glu Tyr
    690                 695                 700

Thr Thr Trp Cys Pro Ile Ser Thr Thr Glu Ser Arg Gln Gln Thr Thr
705                 710                 715                 720

Leu Val Thr Val Thr Ser Cys Glu Ser Gly Val Cys Ser Glu Thr Ala
                725                 730                 735

Ser Pro Ala Ile Val Ser Thr Ala Thr Ala Thr Val Asn Asp Val Val
            740                 745                 750

Thr Val Tyr Pro Thr Trp Arg Pro Gln Thr Ala Asn Glu Glu Ser Val
        755                 760                 765

Ser Ser Lys Met Asn Ser Ala Thr Gly Glu Thr Thr Thr Asn Thr Leu
    770                 775                 780

Ala Ala Glu Thr Thr Thr Asn Thr Val Ala Ala Glu Thr Ile Thr Asn
785                 790                 795                 800

Thr Gly Ala Ala Glu Thr Lys Thr Val Val Thr Ser Ser Leu Ser Arg
                805                 810                 815

Ser Asn His Ala Glu Thr Gln Thr Ala Ser Ala Thr Asp Val Ile Gly
            820                 825                 830

His Ser Ser Ser Val Val Ser Val Ser Glu Thr Gly Asn Thr Lys Ser
        835                 840                 845

Leu Thr Ser Ser Gly Leu Ser Thr Met Ser Gln Gln Pro Arg Ser Thr
    850                 855                 860

Pro Ala Ser Ser Met Val Gly Tyr Ser Thr Ala Ser Leu Glu Ile Ser
865                 870                 875                 880

Thr Tyr Ala Gly Ser Ala Thr Ala Tyr Trp Pro Val Val Val
                885                 890

(2) INFORMATION FOR SEQ ID NO: 23:

     (i) SEQUENCE CHARACTERISTICS:
          (A) LENGTH: 30 base pairs
          (B) TYPE: nucleic acid
          (C) STRANDEDNESS: single
          (D) TOPOLOGY: linear

    (ii) MOLECULE TYPE: DNA (genomic)

   (vii) IMMEDIATE SOURCE:
          (B) CLONE: primer pcrflo1

    (xi) SEQUENCE DESCRIPTION: SEQ ID NO: 23:

GAATTCGCTA GCAATTATGC TGTCAGTACC

(2) INFORMATION FOR SEQ ID NO: 24:

     (i) SEQUENCE CHARACTERISTICS:
          (A) LENGTH: 24 base pairs
          (B) TYPE: nucleic acid
          (C) STRANDEDNESS: single
          (D) TOPOLOGY: linear

    (ii) MOLECULE TYPE: DNA (genomic)

(vii) IMMEDIATE SOURCE:
(B) CLONE: Part non-coding sequence FLO1

(xi) SEQUENCE DESCRIPTION: SEQ ID NO: 24:

AGTGGTACTG ACAGCATAAT TTGA 24

(2) INFORMATION FOR SEQ ID NO: 25:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: Part coding sequence FLO1

(xi) SEQUENCE DESCRIPTION: SEQ ID NO: 25:

AATAAAATTC GCGTTCTTTT TACG 24

(2) INFORMATION FOR SEQ ID NO: 26:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(vii) IMMEDIATE SOURCE:
(B) CLONE: primer pcrflo2

(xi) SEQUENCE DESCRIPTION: SEQ ID NO: 26:

GAGCTCAAGC TTCGTAAAAA GAACGCGAAT T 31

## Description

The present invention is in the field of conversion processes using immobilized enzymes, produced by genetic engineering.

### Background of the invention

In the detergent, personal care and food products industry there is a strong trend towards natural ingredients of these products and to environmentally acceptable production processes. Enzymic conversions are very important for fulfilling these consumer demands, as these processes can be completely natural. Moreover enzymic processes are very specific and consequently will produce minimum amounts of waste products. Such processes can be carried out in water at mild temperatures and atmospheric pressure. However enzymic processes based on free enzymes are either quite expensive due to the loss of enzymes or require expensive equipment, like ultra-membrane systems to entrap the enzyme.

Alternatively enzymes can be immobilized either physically or chemically. The latter method has often the disadvantage that coupling is carried out using non-natural chemicals and in processes that are not attractive from an environmental point of view. Moreover chemical modification of enzymes is nearly always not very specific, which means that coupling can affect the activity of the enzyme negatively.

Physical immobilization can comply with consumer demands, however also physical immobilization may affect the activity of the enzyme in a negative way. Moreover, a physically immobilized enzyme is in equilibrium with free enzyme, which means that in continuous reactors, according to the laws of thermodynamics, substantial losses of enzyme are unavoidable.

There are a few publications on immobilization of enzymes to microbial cells (see reference 1). The present invention provides a method for immobilizing enzymes to cell walls of microbial cells in a very precise way. Additionally, the immobilization does not require any chemical or physical coupling step and is very efficient.

Some extracellular proteins are known to have special functions which they can perform only if they remain bound to the cell wall of the host cell. Often this type of protein has a long C-terminal part that anchors it in the cell wall. These C-terminal parts have very special amino acid sequences. A typical example is anchoring via C-terminal sequences enriched in proline (see reference 2). Another mechanism to anchor proteins in cell walls is that the protein has a glycosyl-phosphatidyl-inositol (GPI) anchor (see reference 3) and that the C-terminal part of the protein contains a substantial number of potential serine and threonine glycosylation sites.

O-Glycosylation of these sites gives a rod-like conformation to the C-terminal part of these proteins. Another feature of these manno-proteins is that they seem to be linked to the glucan in the cell wall of lower eukaryotes, as they cannot be extracted from the cell wall with SDS, but can be liberated by glucanase treatment.

### Summary of the invention

The present invention provides a method for immobilizing an enzyme, which comprises the use of recombinant DNA techniques for producing an enzyme or a functional part thereof linked to the cell wall of a host cell, preferably a microbial cell, and whereby the enzyme or functional fragment thereof is localized at the exterior of the cell wall. Preferably the enzyme or the functional part thereof is immobilized by linking to the C-terminal part of a protein that ensures anchoring in the cell wall.

In one embodiment of the invention a recombinant polynucleotide is provided comprising a structural gene encoding a protein providing catalytic activity and at least a part of a gene encoding a protein capable of anchoring in a eukaryotic or prokaryotic cell wall, said part encoding at least the C-terminal part of said anchoring protein. Preferably the polynucleotide further comprises a sequence encoding a signal peptide ensuring secretion of the expression product of the polynucleotide. Such signal peptide can be derived from a glycosyl-phosphatidyl-inositol (GPI) anchoring protein, α-factor, α-agglutinin, invertase or inulinase, α-amylase of *Bacillus*, or a proteinase of lactic acid bacteria. The DNA sequence encoding a protein capable of anchoring in the cell wall can encode α-agglutinin, AGA1, FLO1 or the Major Cell Wall Protein of lower eukaryotes, or a proteinase of lactic acid bacteria. The recombinant polynucleotide is operably linked to a promoter, preferably an inducible promoter. The DNA sequence encoding a protein providing catalytic activity can encode a hydrolytic enzyme, e.g. a lipase, or an oxidoreductase, e.g. an oxidase.

Another embodiment of the invention relates to a recombinant vector comprising a polynucleotide as described above. If such vector contains a DNA sequence encoding a protein providing catalytic activity, which protein exhibits said catalytic activity when present in a multimeric form, said vector can further comprise a second polynucleotide comprising a structural gene encoding the same protein providing catalytic activity combined with a sequence encoding a signal peptide ensuring secretion of the expression product of said second polynucleotide, said second polynucleotide being operably linked to a regulatable promoter, preferably an inducible or repressible promoter.

A further embodiment of the invention relates to a chimeric protein encoded by a polynucleotide as described above.

Still another embodiment is a host cell, preferably a microorganism, containing a polynucleotide as described above or a vector as described above. If the protein providing catalytic activity exhibits said catalytic activity when present in a multimeric form, said host cell or microorganism can further comprise a second polynucleotide comprising a structural gene encoding the same protein providing catalytic activity combined with a sequence encoding a signal peptide ensuring secretion of the expression product of said second polynucleotide, said second polynucleotide being operably linked to a regulatable promoter, preferably an inducible or repressible promoter, and said second polynucleotide being present either in another vector or in the chromosome of said microorganism. Preferably the host cell or microorganism has at least one of said polynucleotides integrated in its chromosome. As a result of culturing such host cell or microorganism the invention provides a host cell, preferably a microorganism, having a protein as described above immobilized on its cell wall. The host cell or microorganism can be a lower eukaryote, in particular a yeast.

The invention also provides a process for carrying out an enzymatic process by using an immobilized catalytically active protein, wherein a substrate for said catalytically active protein is contacted with a host cell or microorganism according to the invention.

### Brief Description of the Figures

Figure 1: DNA sequence of the 6057 bp *Hin*dIII fragment containing the complete AGα1 gene of *S. cerevisiae* (see SEQ ID NO: 1 and 2). The position of the unique *Nhe*I site and the *Hin*dIII site used for the described constructions is specified in the header.
Figure 2: Schematic presentation of the construction of pUR2969. The restriction sites for endonucleases used are shown. Abbreviations used: AG-alpha-1: Gene expressing α-agglutinin from *S*. *cerevisiae*
   amp: β-lactamase resistance gene
   PGKp: phosphoglyceratekinase promoter
   PGKt: terminator of the same gene.
Figure 3: α-Galactosidase activity of *S. cerevisiae* MT302/1C cells and culture fluid transformed with pSY13 during batch culture:
   A: U/l α-galactosidase per time; the OD₅₃₀ is also shown
   B: α-galactosidase activity of free and bond enzyme expressed in U/OD₅₃₀.
Figure 4: α-Galactosidase activity of *S. cerevisiae* MT302/1C cells and culture fluid transformed with pUR2969 during batch culture:
   A: U/l α-galactosidase per time; the OD₅₃₀ is also shown
   B: α-galactosidase activity of free and bond enzyme expressed in U/OD₅₃₀.
Figure 5: Western analysis with anti α-galactosidase serum of extracellular fractions of cells of exponential phase (OD₅₃₀=2). The analyzed fractions are equivalent to 4 mg cell walls, (fresh weight):
   A: MT302/1C expressing α-galactosidase,
      lane 1, growth medium
      lane 2, SDS extract of isolated cell walls
      lane 3, glucanase extract of SDS extracted cell walls;
   B: MT302/1C expressing α-Gal-AGα1 fusion protein,
      lane 1, growth medium
      lane 2, SDS extract of isolated cell walls
      lane 3, glucanase extract of SDS-extracted cell walls
      lane 4: Endo-H treated glucanase extract.

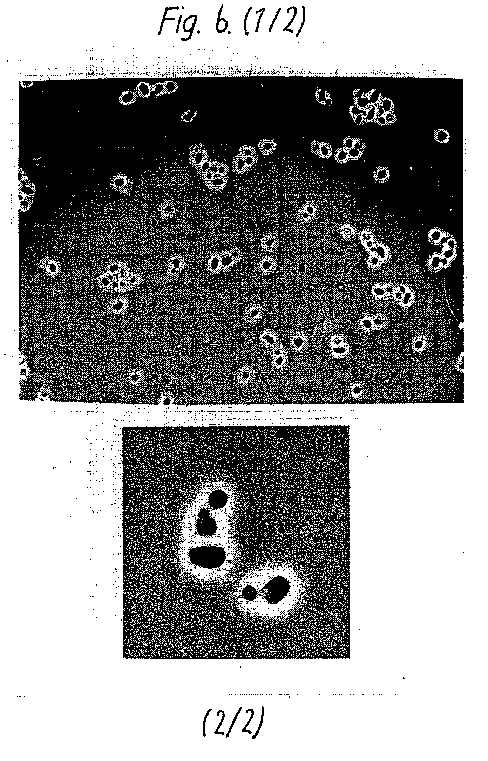

Figure 6: Immunofluorescent labelling (anti α-galactosidase) of MT302/1C cells in the exponential phase (OD₅₃₀=2) expressing the α-Gal-α-agglutinin fusion protein. Phase micrograph of intact cells A: overview B: detail.

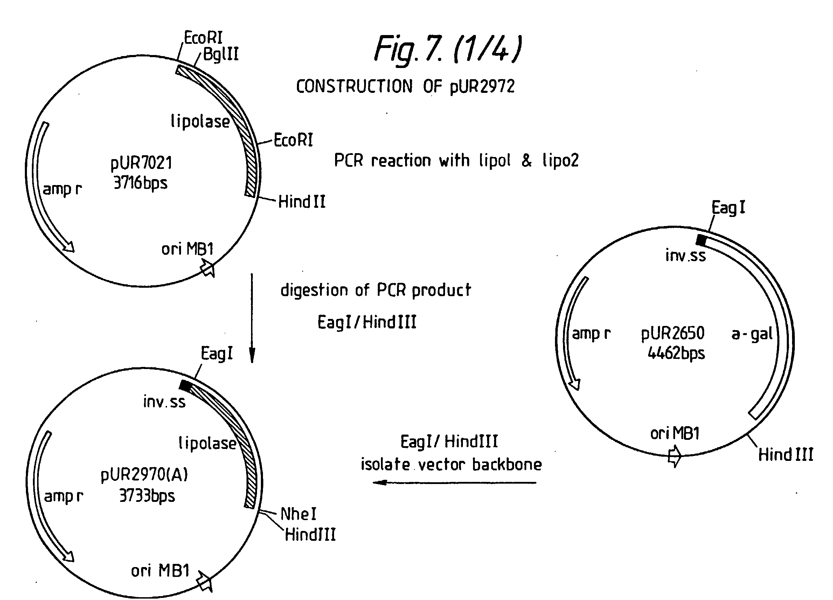

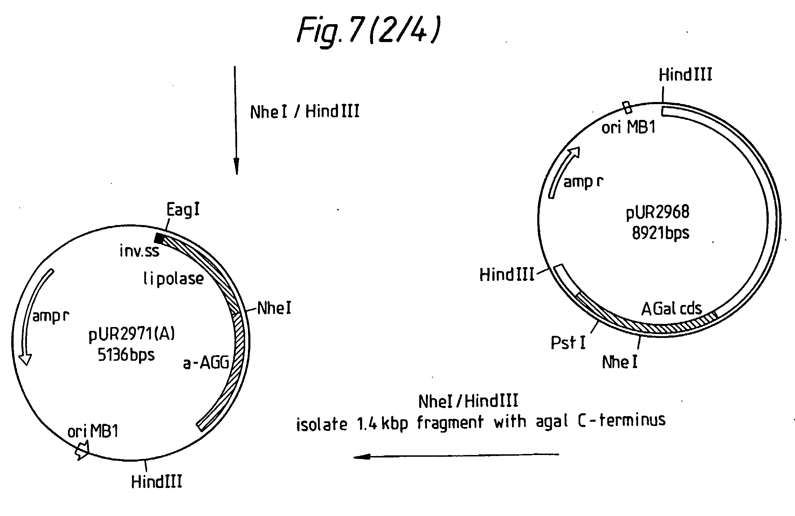

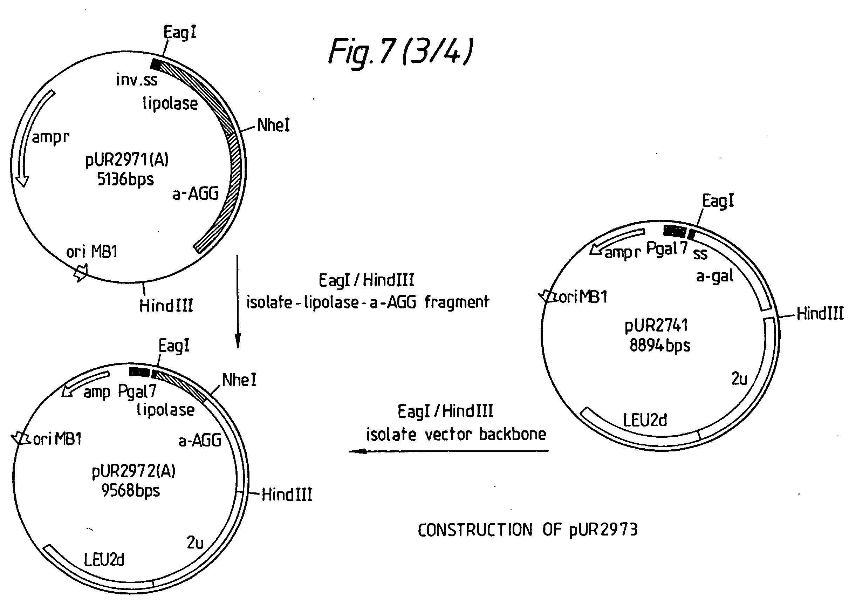

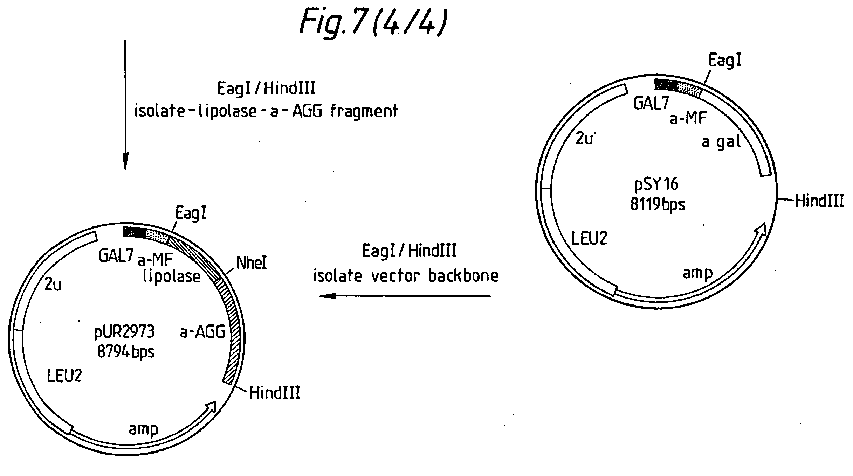

Figure 7: Schematic presentation of the construction of pUR2970A, pUR2971A, pUR2972A, and pUR2973. The restriction sites for endonucleases used are indicated in the figure. PCR oligonucleotide sequences are mentioned in the text. Abbreviations used: AGa1 cds: coding sequence of α-agglutinin
   a-AGG=AGa1: Gene expressing α-agglutinin from *S*. *cerevisiae*
   amp: β-lactamase resistance gene Pgal7=GAL7: *GAL7* promoter
   lipolase: lipase gene of *Humicola* invSS: *SUC2* signal sequence
   a-MF: prepro-α-mating factor sequence a-gal: α-galactosidase gene
   LEU2d : truncated promoter of *LEU2* gene;
   LEU2 : *LEU2* gene with complete promoter sequence.

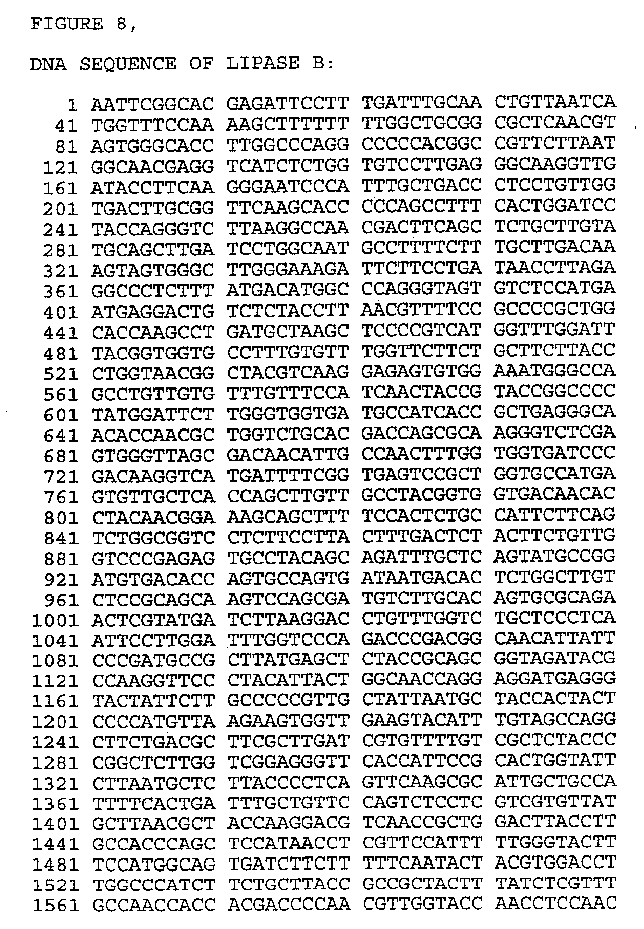

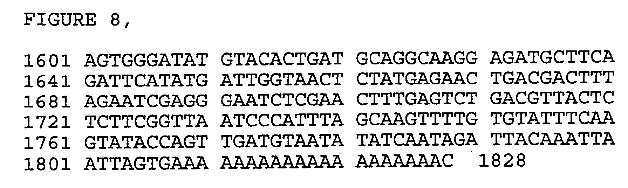

Figure 8: DNA sequence of a fragment containing the complete coding sequence of lipase B of *Geotrichum candidum* strain 335426 (see SEQ ID NO: 11 and 12). The sequence of the mature lipase B starts at nucleotide 97 of the given sequence. The coding sequence starts at nucleotide 40 (ATG).

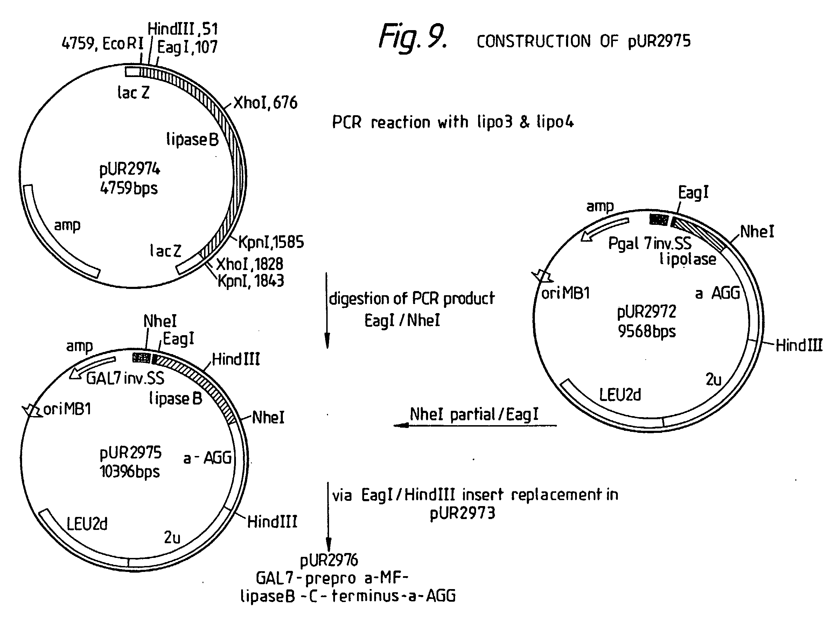

Figure 9: Schematic presentation of the construction of pUR2975 and pUR2976. The restriction sites for endonucleases used are shown. Abbreviations used:
   a-AGG: Gene expressing α-agglutinin from *S*. *cerevisiae*
   amp: β-lactamase resistance gene Pgal7=GAL7: GAL7 promoter
   invSS: *SUC2* signal sequence a-MF: prepro-α-mating factor sequence
   LEU2d: truncated promoter *LEU2* gene lipolase: lipase gene of *Humicola*
   lipaseB: lipaseB gene of *Geotrichum candidum.*

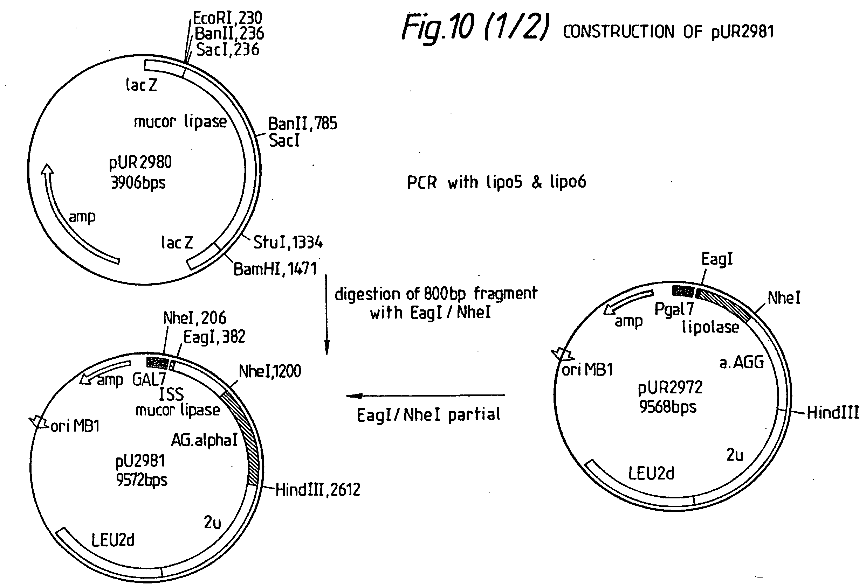

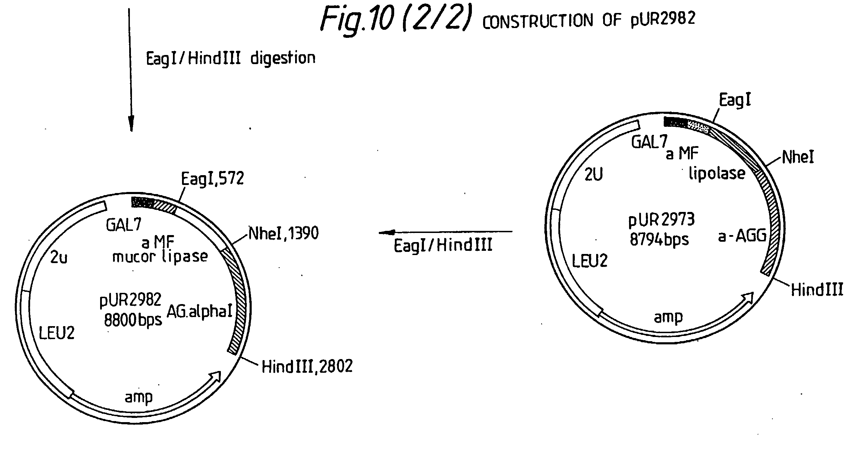

Figure 10: Schematic presentation of the construction of pUR2981 and pUR2982. The restriction sites for endonucleases used are shown. Abbreviations used:
   a-AGG=AG-alpha 1: Gene expressing α-agglutinin from *S*. *cerevisiae*
   mucor lipase: lipase gene of *Rhizomucor miehei* 2u: 2µm sequence
   Pgal7=GAL7: *GAL7* promoter invSS: *SUC2* signal sequence
   a-MF: prepro-α-mating factor sequence lipolase: lipase gene of *Humicola*
   amp: β-lactamase resistance gene; LEU2d: truncated promoter *LEU2* gene
   LEU2 : *LEU2* gene with complete promoter sequence.

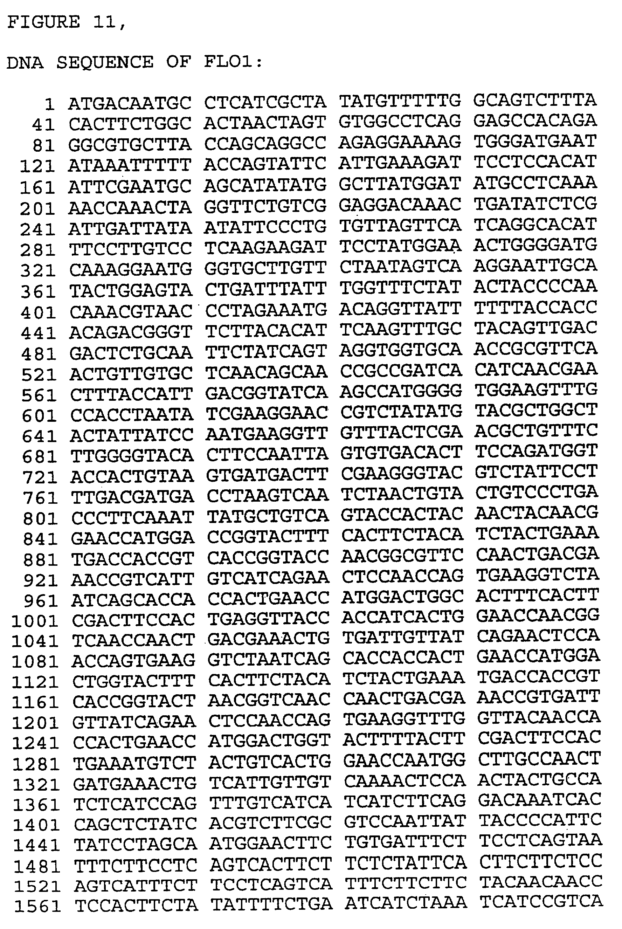

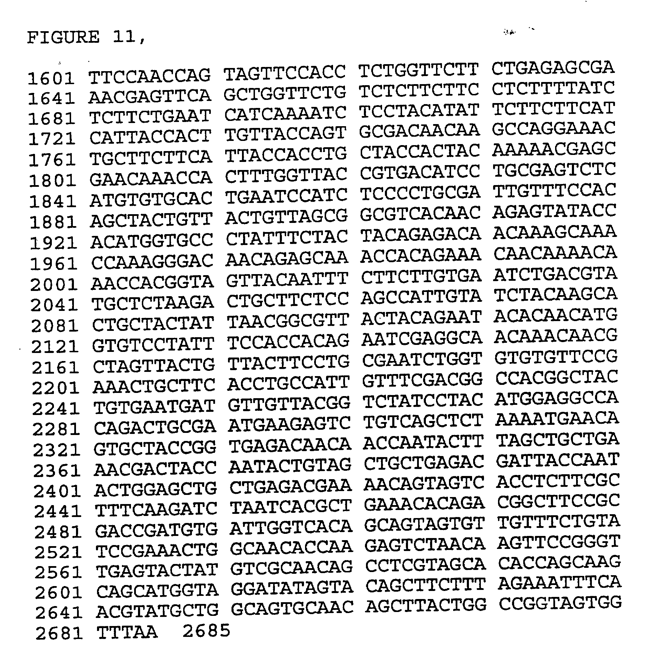

Figure 11: DNA sequence (2685 bases) of the 894 amino acids coding part of the *FLO1* gene (see SEQ ID NO: 21 and 22), the given sequence starts with the codon for the first amino acid and ends with the stop codon.

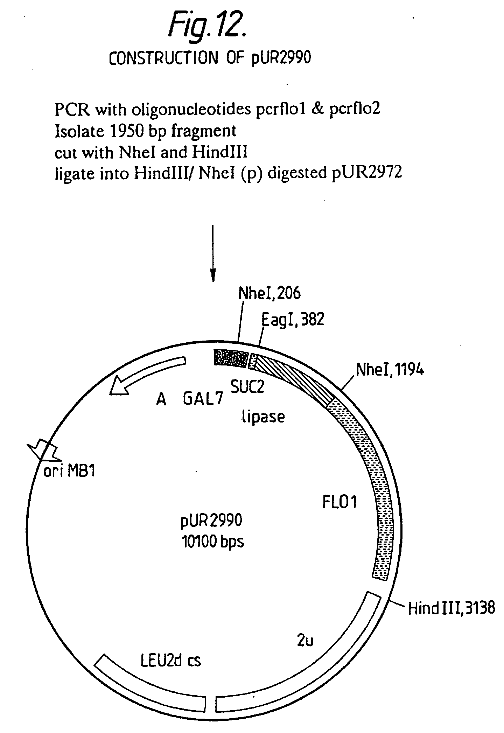

Figure 12: Schematic presentation of plasmid pUR2990. Some restriction sites for endonucleases relevant for the given cloning procedure are shown.

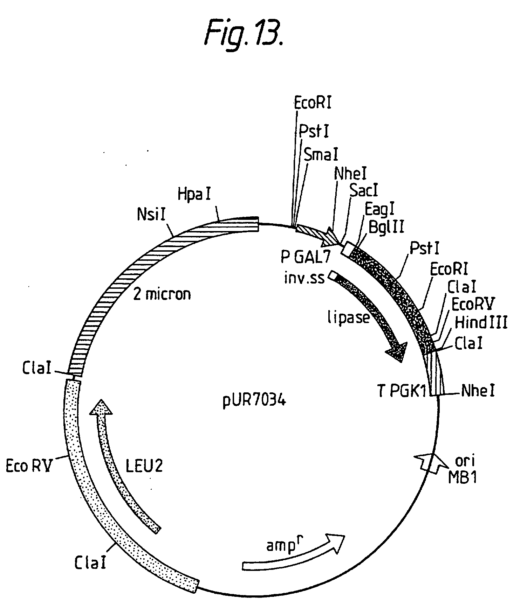

Figure 13: Schematic presentation of plasmid pUR7034.

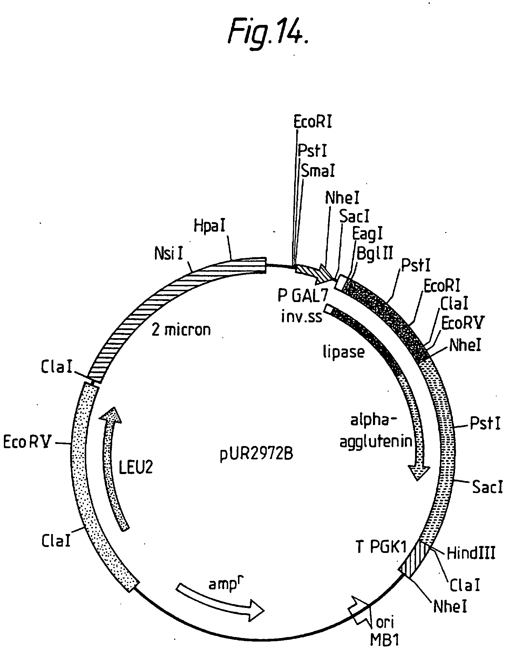

Figure 14: Schematic presentation of plasmid pUR2972B.

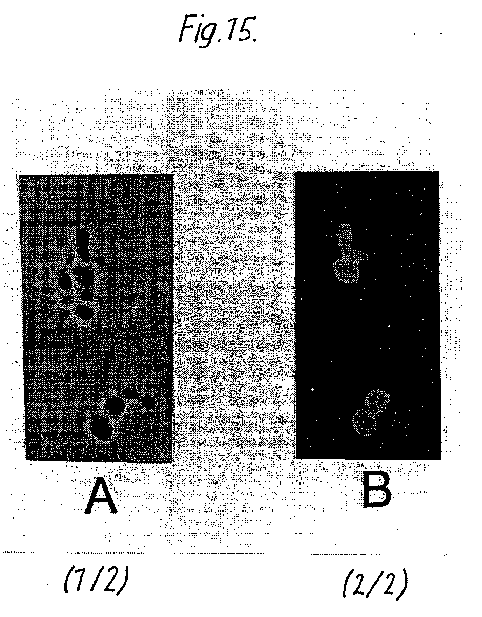

Figure 15: Immunofluorescent labelling (anti-lipolase) of SU10 cells in the exponential phase (OD₅₃₀=0.5) expressing the lipolase/-α-agglutinin fusion protein. A: phase micrograph B: matching fluorescent micrograph

### Detailed description of the invention

The present invention provides a method for immobilizing an enzyme, comprising immobilizing the enzyme or a functional part thereof to the cell wall of a host cell, preferably a microbial cell, using recombinant DNA techniques. In particular, the C-terminal part of a protein that ensures anchoring in the cell wall is linked to an enzyme or the functional part of an enzyme, in such a way that the enzyme is localized on or just above the cell surface. In this way immobilized enzymes are obtained on the surface of cells. The linkage is performed at gene level and is characterized in that the structural gene coding for the enzyme is coupled to at least part of a gene encoding an anchor-protein in such a way that in the expression product the enzyme is coupled at its C-terminal end to the C-terminal part of an anchor-protein. The chimeric enzyme is preferably preceded by a signal sequence that ensures efficient secretion of the chimeric protein.

Thus the invention relates to a recombinant polynucleotide comprising a structural gene encoding a protein providing catalytic activity and at least a part of a gene encoding a protein capable of anchoring in a eukaryotic or prokaryotic cell wall, said part encoding at least the C-terminal part of said anchoring protein. The length of the C-terminal part of the anchoring protein may vary. Although the entire structural protein could be used, it is preferred that only a part is used, leading to a more efficient exposure of the enzyme protein in the medium surrounding the cell. The anchoring part of the anchoring protein should preferably be entirely present. As an example, about the C-terminal half of the anchoring protein could be used. Preferably, the polynucleotide further comprises a sequence encoding a signal peptide ensuring secretion of the expression product of the polynucleotide. The signal peptide can be derived from a GPI anchoring protein, α-factor, α-agglutinin, invertase or inulinase, α-amylase of *Bacillus,* or a proteinase of lactic acid bacteria.

The protein capable of anchoring in the cell wall is preferably selected form the group of α-agglutinin, AGA1, FLO1 (flocculation protein) or the Major Cell Wall Protein of lower eukaryotes, or a proteinase of lactic acid bacteria. The polynucleotide of the invention is preferably operably linked to a promoter, preferably a regulatable promoter, especially an inducible promoter.

The invention also relates to a recombinant vector containing the polynucleotide as described above, and to a host cell containing this polynucleotide, or this vector.

In a particular case, wherein the protein providing catalytic activity exhibits said catalytic activity when present in a multimeric form, such as may be the case with oxidoreductases, dimerisation or multimerisation of the monomers might be a prerequisite for activity. The vector and/or the host cell can then further comprise a second polynucleotide comprising a structural gene encoding the same protein providing catalytic activity combined with a sequence encoding a signal peptide ensuring secretion of the expression product of said second polynucleotide, said second polynucleotide being operably linked to a regulatable promoter, preferably an inducible or repressible promoter. Expression and secretion of the second polynucleotide after expression and secretion of the first polynucleotide will then result in the formation of an active multimer on the exterior of the cell wall.

The host cell or microorganism preferably contains the polynucleotide described above, or at least one of said polynucleotides in the case of a combination, integrated in its chromosome.

The present invention relates in particular to lower eukaryotes like yeasts that have very stable cell walls and have proteins that are known to be anchored in the cell wall, e.g. α-agglutinin or the product of gene *FLO1.* Suitable yeasts belong to the genera *Candida, Debaryomyces*, *Hansenula*, *Kluyveromyces*, *Pichia* and *Saccharomyces.*

Also fungi, especially *Aspergillus*, *Penicillium* and *Rhizopus* can be used. For certain applications also prokaryotes are applicable.

For yeasts the present invention deals in particular with genes encoding chimeric enzymes consisting of:
a. the signal sequence e.g. derived from the α-factor-, the invertase-, the α-agglutinin- or the inulinase genes;
b. structural genes encoding hydrolytic enzymes such as α-galactosidase, proteases, peptidases, pectinases, pectylesterase, rhamnogalacturonase, esterases and lipases, or non-hydrolytic enzymes such as oxidases; and
c. the C-terminus of typically cell wall bound proteins such as α-agglutinin (see reference 4), AGA1 (see reference 5) and FLO1 (see the non-prior published reference 6).

The expression of these genes can be under the control of a constitutive promoter, but more preferred are regulatable, i.e. repressible or inducible promoters such as the *GAL7* promoter for *Saccharomyces,* or the inulinase promoter for *Kluyveromyces* or the methanol-oxidase promoter for *Hansenula.*

Preferably the constructs are made in such a way that the new genetic information is integrated in a stable way in the chromosome of the host cell.

The invention further relates to a host cell, in particular a microorganism, having the chimeric protein described above immobilized on its cell wall. It further concerns the use of such microorganisms for carrying out an enzymatic process by contacting a substrate for the enzyme with the microorganism. Such a process may be carried out e.g. in a packed column, wherein the microorganisms may be supported on solid particles, or in a stirred reactor. The reaction may be aqueous or non-aqueous. Where necessary, additives necessary for the performance of the enzyme, e.g. a co-factor, may be introduced in the reaction medium.

After repeated usage of the naturally immobilized enzyme system in processes, the performance of the system may decrease. This is caused either by physical denaturation or by chemical poisoning or detachment of the enzyme. A particular feature of the present invention is that after usage the system can be recovered from the reaction medium by simple centrifugation or membrane filtration techniques and that the thus collected cells can be transferred to a recovery medium in which the cells revive quickly and concomitantly produce the chimeric protein, thus ensuring that the surface of the cells will be covered by fully active immobilized enzyme. This regeneration process is simple and cheap and therefore will improve the economics of enzymic processes and may result in a much wider application of processes based on immobilized enzyme systems.

However, by no means the present invention is restricted to the reusability of the immobilized enzymes.

The invention will be illustrated by the following examples without the scope of the invention being limited thereto.

### EXAMPLE 1 Immobilized a-galactosidase/α-agglutinin on the surface of S. cerevisiae.

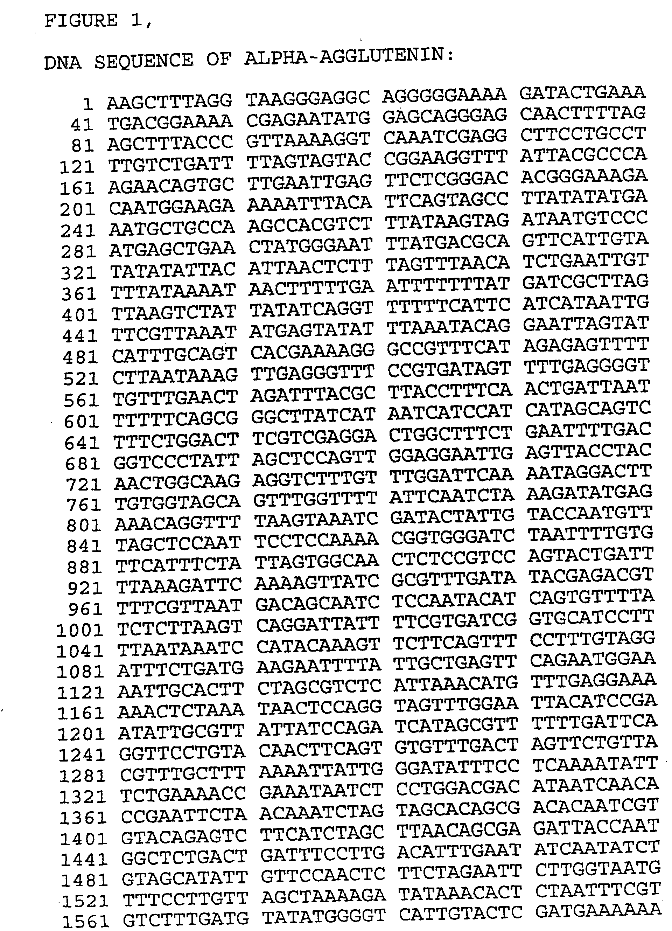

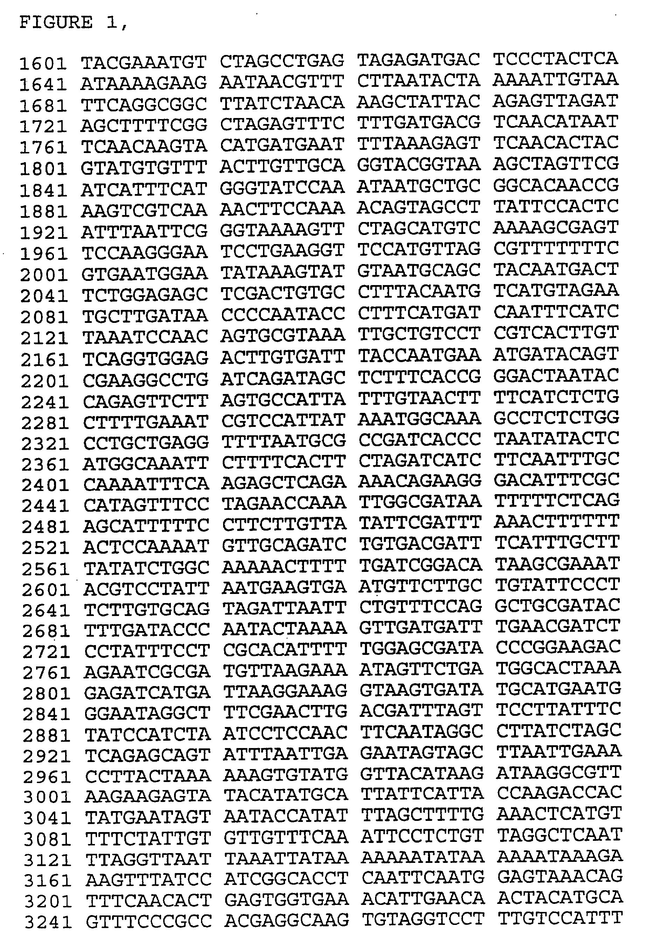

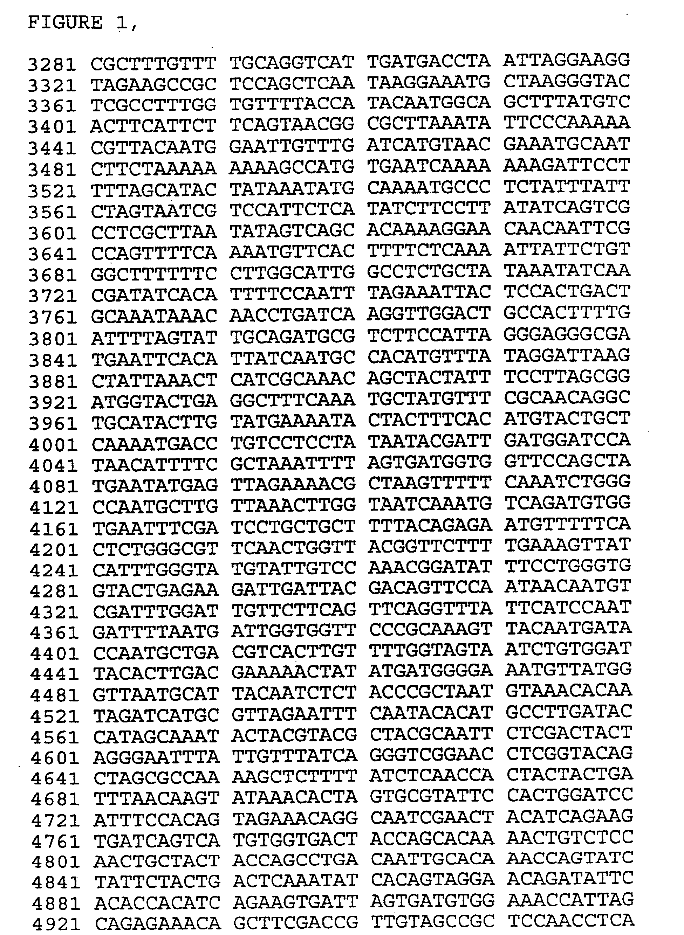

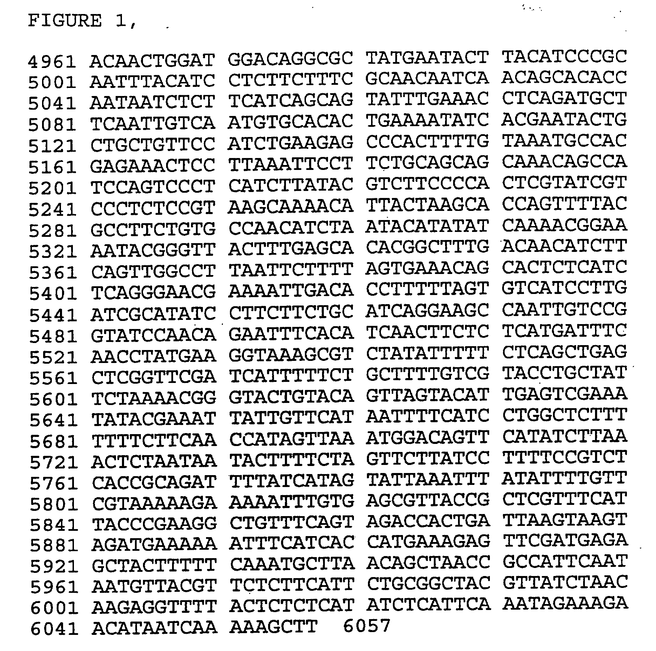

The gene encoding α-agglutinin has been described by Lipke *et al.* (see reference 4). The sequence of a 6057 bp *Hin*dIII insert in pTZ18R, containing the whole AGα1 gene is given in Figure 1. The coding sequence expands over 650 amino acids, including a putative signal sequence starting at nucleotide 3653 with ATG. The unique *Nhe*I site cuts the DNA at position 988 of the given coding sequence within the coding part of amino acid 330, thereby separating the α-agglutinin into an N-terminal and a C-terminal part of about same size.

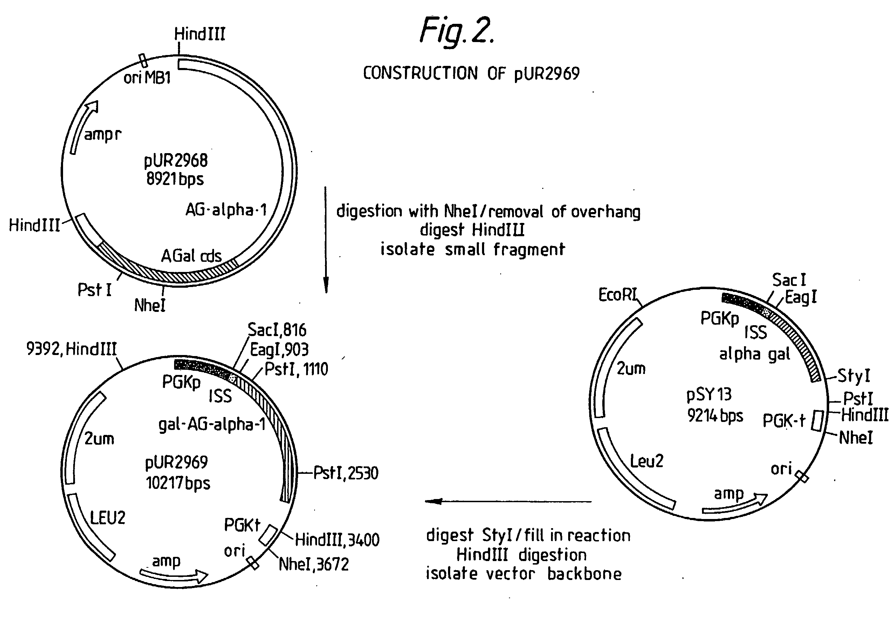

Through digestion of pUR2968 (see Figure 2) with *Nhe*I/*Hin*dIII a 1.4 kb fragment was released, containing the sequence information of the putative cell wall anchor. For the fusion to α-galactosidase the plasmid pSY16 was used, an episomal vector based on YEplac 181, harbouring the α-galactosidase sequence preceded by the *SUC2* invertase signal sequence and placed between the constitutive *PGK* promoter and *PGK* terminator. The *Sty*I site, present in the last nine base-pairs of the open reading frame of the α-galactosidase gene, was ligated to the *Nhe*I site of the *AGα1* gene fragment. To ensure the in frame fusion, the *Sty*I site was filled in and the 5' overhang of the *Nhe*I site was removed, prior to ligation into the *Sty*I/ *Hin*dIII digested pSY13 (see Figure 2).

To verify the correct assembly of the new plasmid, the shuttle vector was transformed into E. *coli* JM109 (*recA1 supE44 endA1 hsdR17 gyrA96 relA1 thi ▲*(*lac-proAB*) *F'* [*traD36 proAB*^{*+*} *lacI*^{q} *lacZ*▲M15]) (see reference 7) by the transformation protocol described by Chung *et al.* (see reference 8). One of the positive clones, designated pUR2969, was further characterized, the DNA isolated and purified according to the Quiagen protocol and subsequently characterized by DNA sequencing. DNA sequencing was mainly performed as described by Sanger *et al.* (see reference 9), and

Hsiao (see reference 10), here with the Sequenase version 2.0 kit from United States Biochemical Company, according to the protocol with T7 DNA polymerase (Amersbam International plc) and [³⁵S]dATPαS (Amersham International plc: 370 MBq/ml; 22 TBq/mmol).

This plasmid was then transformed into *S. cerevisiae* strain MT302/1C according to the protocol from Klebe *et al.* (see reference 11).

Yeast transformants were selected on selective plates, lacking leucine, on with 40 µl (20mg/ml DMF). X-α-Gal (5-bromo-4-chloro-3-indolyl-α-D-glucose, Boehringer Mannheim) was spread, to directly test for α-galactosidase activity (see reference 12). To demonstrate the expression, secretion, localization and activity of the chimeric protein the following analyses were performed:

### 1. Expression and secretion

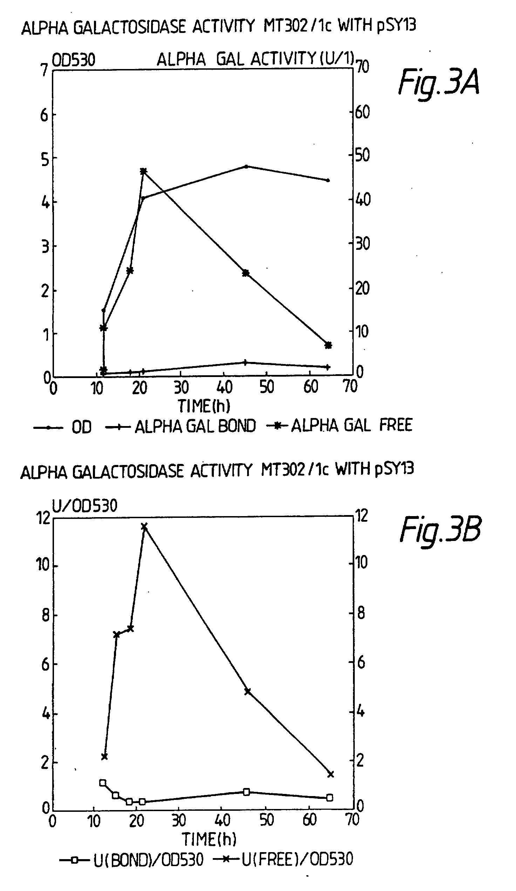

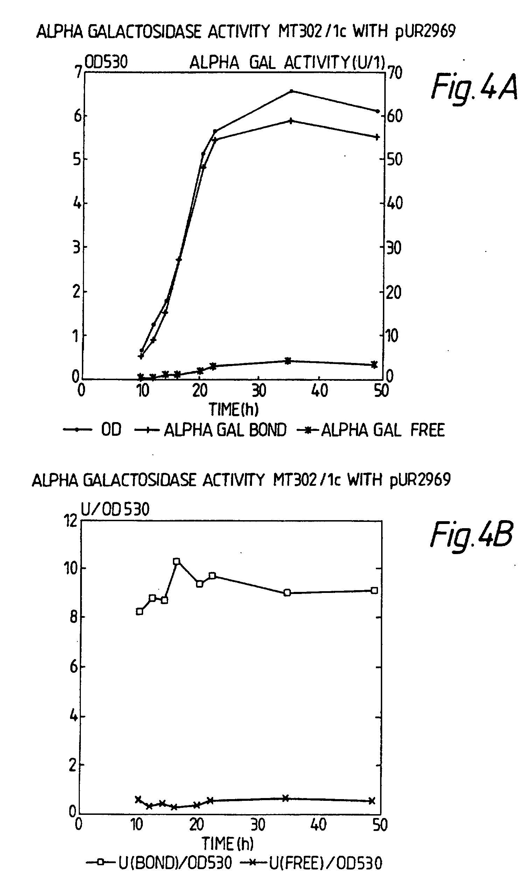

*S. cerevisiae* strain MT302/1C was transformed with either plasmid pSY13 containing the α-galactosidase gene of *Cyamopsis tetragonoloba* or plasmid pUR2969 containing the α-galactosidase/α-agglutinin fusion construct. During batch culture α-galactosidase activities were determined for washed cells and growth medium. The results are given in Figure 3 and Figure 4. The α-galactosidase expressed from yeast cells containing plasmid pSY13 was almost exclusively present in the growth medium (Figure 3A), whereas the α-galactosidase-α-agglutinin fusion protein was almost exclusively cell associated (Figure 4A). Moreover, the immobilized, cell wall-associated, α-galactosidase-α-agglutinin fusion enzyme had retained the complete activity over the whole incubation time, while the secreted and released enzyme lost about 90% of the activity after an incubation of 65 hours. This indicates, that the immobilization of the described enzyme into the cell wall of yeast protects the enzyme against inactivation, presumably through proteinases, and thereby increases the stability significantly. Further insight into the localization of the different gene products was obtained by Western analysis. Therefore, cells were harvested by centrifugation and washed in 10 mM Tris.HCl, pH 7.8; 1 mM PMSF at 0°C and all subsequent steps were performed at the same temperature. Three ml isolation buffer and 10 g of glass beads were added per gram of cells (wet weight). The mixture was shaken in a Griffin shaker at 50% of its maximum speed for 30 minutes. The supernatant was isolated and the glass beads were washed with 1 M NaCl and 1 mM PMSF until the washes were clear. The supernatant and the washes were pooled. The cell walls were recovered by centrifugation and were subsequently washed in 1 mM PMSF.

Non-covalently bound proteins or proteins bound through disulphide bridges were released from cell walls by boiling for 5 minutes in 50 mM Tris.HCl, pH 7.8; containing 2 % SDS, 100 mM EDTA and 40 mM β-mercaptoethanol. The SDS-extracted cell walls were washed several times in 1 mM PMSF to remove SDS. Ten mg of cell walls (wet weight) were taken up in 20 1 100 mM sodium acetate, pH 5.0, containing 1 mM PMSF. To this, 0.5 mU of the β-1,3-glucanase (Laminarase; Sigma L5144) was used as a source of β-1,3-glucanase) was added followed by incubation for 2 hours at 37 °C. Subsequently another 0.5 mU of β-1,3-glucanase was added, followed by incubation for another 2 hours at 37 °C

Proteins were denatured by boiling for 5 minutes preceding Endo-H treatment. Two mg of protein were incubated in 1 ml 50 mM potassium phosphate, pH 5.5, containing 100 mM β-mercaptoethanol and 0.5 mM PMSF with 40 mU Endo-H (Boehringer) for 48 hours at 37 °C. Subsequently 20 mU Endo-H were added followed by 24 hours of incubation at 37 °C.

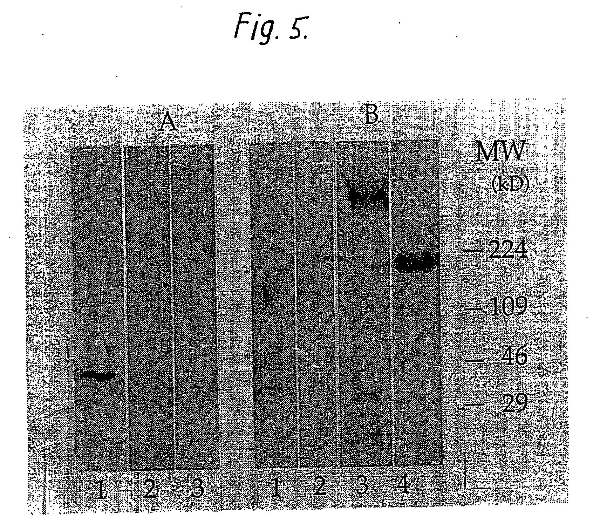

Proteins were separated by SDS-PAGE according to Laemmli (see reference 13) in 2.2.-20% gradient gels. The gels were blotted by electrophoretic transfer onto Immobilon polyvinylidene-difluoride membrane (Millipore) as described by Towbin *et al.* (see reference 14). In case of highly glycosylated proteins a subsequently mild periodate treatment was performed in 50 mM periodic acid, 100 mM sodium acetate, pH 4.5, for several hours at 4 °C. All subsequent incubations were carried out at room temperature. The blot was blocked in PBS, containing 0.5% gelatine and 0.5% Tween-20, for one hour followed by incubation for 1 hour in probe buffer (PBS, 0.2% gelatine, 0.1% Tween-20) containing 1:200 diluted serum. The blot was subsequently washed several times in washing buffer (PBS; 0.2% gelatine; 0.5% Tween-20) followed by incubation for 1 hour in probe-buffer containing ¹²⁵I-labelled protein A (Amersham). After several washes in washing buffer, the blot was air-dried, wrapped in Saran (Dow) and exposed to X-omat S film (Kodak) with intensifying screen at -70 °C. An Omnimedia 6cx scanner and the Adobe Photoshop programme were used to quantify the amount of labelled protein. The results of the various protein isolation procedures from both transformants are given in Figure 5. While for the transformants comprising the pSY13 plasmid the overall mass of the enzyme was localized in the medium, with only minor amounts of enzyme more entrapped than bond in the cell wall (Figure 5A) -which could completely be removed by SDS extraction- the fusion protein was tightly bound to the cell wall; with only small amounts of α-galactosidase/α-agglutinin delivered into the surrounding culture fluid or being SDS extractable. In contrast to the laminarinase extraction of cell walls from cells expressing the free α-galactosidase, where no further liberation of any more enzyme was observed, identical treatment of fusion enzyme expressing cells released the overall bulk of the enzyme. This indicates that the fusion protein is intimately associated with the cell wall glucan in *S. cerevisiae,* like α-agglutinin, while α-galactosidase alone is not. The subsequently performed EndoH treatment showed a heavy glycosylation of the fusion protein, a result, entirely in agreement with the described extended glycosylation of the C-terminal part of α-agglutinin.

### 2. Localization

Immunofluorescent labelling with anti-α-galactosidase serum was performed on intact cells to determine the presence and distribution of α-galactosidase/α-agglutinin fusion protein in the cell wall. Immunofluorescent labelling was carried out without fixing according to Watzele *et al.* (see reference 15). Cells of OD₅₃₀ = 2 were isolated and washed in TBS (10 mM Tris.HCl, pH 7.8, containing 140 mM NaCI, 5 mM EDTA and 20 µg/ml cycloheximide). The cells were incubated in TBS + anti-α-galactosidase serum for 1 hour, followed by several washings in TBS. A subsequent incubation was carried out with FITC-conjugated anti-rabbit IgG (Sigma) for 30 minutes. After washing in TBS, cells were taken up in 10 mM Tris.HCl, pH 9.0, containing 1 mg/ml p-phenylenediamine and 0.1 % azide and were photographed on a Zeiss 68000 microscope. The results of these analysis are given in Figure 6, showing clearly that the chimeric α-galactosidase/α-agglutinin is localized at the surface of the yeast cell. Buds of various sizes, even very small ones very uniformly labelled, demonstrates that the fusion enzyme is continuously incorporated into the cell wall throughout the cell cycle and that it instantly becomes tightly linked.

### 3. Activity

To quantitatively assay α-galactosidase activity, 200 µl samples containing 0.1 M sodium-acetate, pH 4.5 and 10 mM p-nitrophenyl-α-D-galactopyranoside (Sigma) were incubated at 37 °C for exactly 5 minutes. The reaction was stopped by addition of 1 ml 2% sodium carbonate. From intact cells and cell walls, removed by centrifugation and isolated and washed as described, the α-galactosidase activity was calculated using the extinction coefficient of p-nitrophenol of 18.4 cm²/mole at 410 nm.

One unit was defined as the hydrolysis of 1 µmole substrate per minute at 37 °C.

**Table 1.**

| Distribution of free and immobilized α-galactosidase activity in yeast cells | | | |
|---|---|---|---|
| | α-Galactosidase activity (U/g F.W. cells) | | |
| Expressed protein | Growth medium | Intact cells | Isolated cell walls |
| α-galactosidase | 14.7 | 0.37 | 0.01 |
| αGal/αAGG fusion protein | 0.54 | 13.3 | 10.9 |
| Transformed MT302/1C cells were in exponential phase (OD₅₃₀=2). One unit is defined as the hydrolysis of 1 µmole of p-nitrophenyl-α-D-galactopyranoside per minute at 37 °C. | | | |

The results are summarized in Table 1. While the overall majority of α-galactosidase was distributed in the culture fluid, most of the fusion product was associated with the cells, primarily with the cell wall. Taking together the results shown in Figures 3 to 6 and in Table 1, it could be calculated that the enzymatic α-galactosidase activity of the chimeric enzyme is as good as that of the free enzyme. Moreover, during stationary phase, the activity of the α-galactosidase in the growth medium decreased, whereas the activity of the cell wall associated α-galactosidase α-agglutinin fusion remained constant, indicating that the cell associated fusion protein is protected from inactivation or proteolytic degradation.

N.B. The essence of this EXAMPLE was published during the priority year by M.P. Schreuder *et al.* (see reference 25).

### EXAMPLE 2A Immobilized Humicola lipase/α-agglutinin on the surface of S. cerevisiae. (inducible expression of immobilized enzyme system)

The construction and isolation of the 1.4 kb *Nhe*I/*Hin*dIII fragment containing the C-terminal part of α-agglutinin has been described in EXAMPLE 1. Plasmid pUR7021 contains an 894 bp long synthetically produced DNA fragment encoding the lipase of *Humicola* (see reference 16 and SEQ ID NO: 7 and 8), cloned into the *Eco*RI/*Hin*dIII restriction sites of the commercially available vector pTZ18R (see Figure 7). For the proper one-step modification of both the 5' end and the 3' end of the DNA part coding for the mature lipase, the PCR technique can be applied. Therefore the DNA oligonucleotides lipo1 (see SEQ ID NO: 3) and lipo2 (see SEQ ID NO: 6) can be used as primers in a standard PCR protocol, generating an 826 bp long DNA fragment with an *Eag*I and a *Hin*dIII restriction site at the ends, which can be combined with the larger part of the *Eag*I/*Hin*dIII digested pUR2650, a plasmid containing the α-galactosidase gene preceded by the invertase signal sequence as described earlier in this specification, thereby generating plasmid pUR2970A (see Figure 7).

PCR oligonucleotides for the in-frame linkage of *Humicola* lipase and the C-terminus of α agglutinin.

### a: PCR oligonucleotides for the transition between SUC2 signal sequence and the N-terminus of lipase.

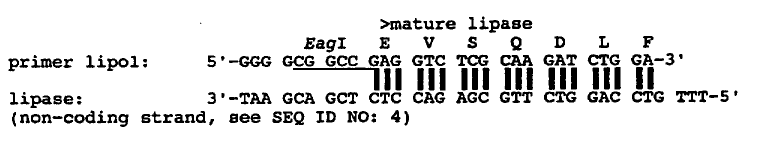
>mature lipase
EagI E V S Q D L F
primer lipo1: 5'-GGG GCG GCC GAG GTC TCG CAA GAT CTG GA-3'
lipase: 3'-TAA GCA GCT CTC CAG AGC GTT CTG GAC CTG TTT-5'
(non-coding strand, see SEQ ID NO: 4)

### b: PCR oligonucleotides for the in frame transition between C-terminus of lipase and C-terminal part of α-agglutinin.

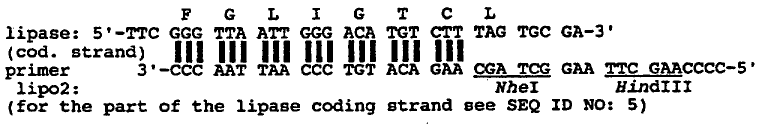
F G L I G T C L
lipase: 5'-TTC GGG TTA ATT GGG ACA TGT CTT TAG TGC GA-3'
(cod. strand)
primer 3'-CCC AAT TAA CCC TGT ACA GAA CGA TCG GAA TTC GAACCCC-5'
lipo2: NheI HindIII
(for the part of the lipase coding strand see SEQ ID NO: 5)

Through the PCR method a *Nhe*I site will be created at the end of the coding sequence of the lipase, allowing the in-frame linkage between the DNA coding for lipase and the DNA coding for the C-terminal part of α-agglutinin. Plasmid pUR2970A can then be digested with *Nhe*I and *Hin*dIII and the 1.4 kb *Nhe*I/*Hin*dIII fragment containing the C-terminal part of α-agglutinin from plasmid pUR2968 can be combined with the larger part of *Nhe*I and *Hin*dIII treated plasmid pUR2970A, resulting in plasmid pUR2971A. From this plasmid the 2.2 kb *Eag*I/*Hin*dIII fragment can be isolated and ligated into the *Eag*I- and *Hin*dIII-treated pUR2741, whereby plasmid pUR2741 is a derivative of pUR2740 (see reference 17), where the second *Eag*I restriction site in the already inactive *Tet* resistance gene was deleted through *Nru*I/*Sal*I digestion. The *Sal*I site was filled in prior to religation. The ligation then results in pUR2972A containing the *GAL7* promoter, the invertase signal sequence, the chimeric lipase/α-agglutinin gene, the 2 µm sequence, the defective *Leu2* promoter and the *Leu2* gene. This plasmid can be used for transforming *S*. *cerevisiae* and the transformed cells can be cultivated in YP medium containing galactose as an inducer without repressing amounts of glucose being present, which causes the expression of the chimeric lipase/α-agglutinin gene.

The expression, secretion, localization and activity of the chimeric lipase/α-agglutinin can be analyzed using similar procedures as given in EXAMPLE 1.

In a similar way variants of *Humicola* lipase, obtained via rDNA techniques, can be linked to the C-terminal part of α-agglutinin, which variants can have a higher stability during (inter)esterification processes.

### EXAMPLE 2B Immobilized Humicola lipase/α-agglutinin on the surface of S. cerevisiae (inducible expression of immobilized enzyme system)

EXAMPLE 2A describes a protocol for preparing a particular construct. Before carrying out the work it was considered more convenient to use the expression vector described in EXAMPLE 1, so that the construction route given in this EXAMPLE 2B differs on minor points from the construction route given in EXAMPLE 2A and the resulting plasmids are not identical to those described in EXAMPLE 2A. However, the essential gene construct comprising the promoter, signal sequence, and the structural gene encoding the fusion protein are the same in EXAMPLES 2A and 2B.

### 1. Construction

The construction and isolation of the 1.4 kb *Nhe*I/*Hin*dIII fragment encoding the C-terminal part of α-agglutinin cell wall protein has been described in EXAMPLE 1. The plasmid pUR7033 (resembling pUR7021 of EXAMPLE 2A) was made by treating the commercially available vector pTZ18R with *Eco*RI and *Hin*dIII and ligating the resulting vector fragment with an 894 bp long synthetically produced DNA *Eco*RI/*Hin*dIII fragment encoding the lipase of *Humicola* (see SEQ ID NO: 7 and 8, and reference 16).

For the fusion of the lipase to the C-terminal, cell wall anchor-comprising domain of α-agglutinin, plasmid pUR7033 was digested with *Eag*I and *Hind*III, and the lipase coding sequence was isolated and ligated into the *Eag*I- and *Hin*dIII-digested yeast expression vector pSY1 (see reference 27), thereby generating pUR7034 (see Figure 13). This is a 2µm episomal expression vector, containing the α-galactosidase gene described in EXAMPLE 1, preceded by the invertase *(SUC2)* signal sequence under the control of the inducible *GAL7* promoter.

Parallel to this digestion, pUR7033 was also digested with *Eco*RV and *Hin*dIII, thereby releasing a 57 bp long DNA fragment, possessing codons. for the last 15 carboxyterminal amino acids. This fragment was exchanged against a small DNA fragment, generated through the hybridisation of the two chemically synthesized deoxyoligonucleotides SEQ ID NO: 9 and SEQ ID NO: 10. After annealing of both DNA strands, these two oligonucleotides essentially reconstruct the rest of the 3' coding sequence of the initial lipase gene, but additionally introduce downstream of the lipase gene a new *Nhe*I restriction site, followed by a *Hin*dIII site in close vicinity, whereby the first three nucleotides of the *Nhe*I site form the codon for the last amino acid of the lipase. The resulting plasmid was designated pUR2970B. Subsequently, this construction intermediate was digested with *Eag*I and *Nhe*I, the lipase encoding fragment was isolated, and, together with the 1.4 kb *Nhe*I/*Hin*dIII fragment of pUR2968 ligated into the *Eag*I- and *Hin*dIII-cut pSY1 vector. The outcome of this 3-point-ligation was called pUR2972B (see Figure 14), the final lipolase-α-agglutinin yeast expression vector.

This plasmid was used for transforming *S*. *cerevisiae* strain SU10 as described in reference 17 and the transformed cells were cultivated in YP medium containing galactose as the inducer without repressing amounts of glucose being present, which causes the expression of the chimeric lipase/α-agglutinin gene.

### 2. Activity

To quantify the lipase activity, two activity measurements with two separate substrates were performed. In both cases, SU10 yeast cells transformed with either plasmid pUR7034 or pSY1 served as control. Therefore, yeast cell transformants containing either plasmid pSY1 or plasmid pUR7034 or plasmid pUR2972B were grown up for 24h in YNB-glucose medium supplied with histidine and uracil, then diluted 1:10 in YP-medium supplied with 5% galactose, and again cultured. After 24h incubation at 30°C, a first measurement for both assays was performed.

The first assay applied was the pH stat method. Within this assay, one unit of lipase activity is defined as the amount of enzyme capable of liberating one micromole of fatty acid per minute from a triglyceride substrate under standard assay conditions (30 ml assay solution containing 38 mM olive oil, considered as pure trioleate, emulsified with 1:1 w/w gum arabic, 20 mM calcium chloride, 40 mM sodium chloride, 5 mM Tris, pH 9.0, 30°C) in a radiometer pH stat apparatus (pHM 84 pH meter, ABU 80 autoburette, TTA 60 titration assembly). The fatty acids formed were titrated with 0.05 N NaOH and the activity measured was based on alkali consumption in the interval between 1 and 2 minutes after addition of putative enzyme batch. To test for immobilized lipase activity, 1 ml of each culture was centrifuged, the supernatant was saved, the pellet was resuspended and washed in 1 ml 1 M sorbitol, subsequently again centrifuged and resuspended in 200µl 1 M sorbitol. From each type of yeast cell the first supernatant and the washed cells were tested for lipase activity.

| A: Lipase activity after 24h (LU/ml) | | |
|---|---|---|
| | cell bound | culture fluid |
| pSY1 | 5.9 | 8.8 |
| pUR7034 | 24.1 | 632.0 |
| pUR2972B-(1) | 18.7 | 59.6 |
| pUR2972B-(2) | 24.6 | 40.5 |

| B: Lipase activity after 48h (LU/ml) | | | |
|---|---|---|---|
| | cell bound | culture fluid | OD660 |
| pSY1 | 6.4 | 4.3 | ⁻40 |
| pUR7034 | 215.0 | 2750.0 | ⁻40 |
| pUR2972B-(1) | 37.0 | 87.0 | ⁻40 |
| pUR2972B-(2) | 34.0 | 82.0 | ⁻40 |

The rest of the yeast cultures was further incubated, and essentially the same separation procedure was done after 48 hours. Dependent on the initial activity measured, the actual volume of the sample measured deviated between 25µl and 150µl.

This series of measurements indicates, that yeast cells comprising the plasmid coding for the lipase-α-agglutinin fusion protein in fact express some lipase activity which is associated with the yeast cell.

An additional second assay was performed to further confirm the immobilization of activity of lipase on the yeast cell surface. Briefly, within this assay, the kinetics of the PNP (=paranitrophenyl) release from PNP-butyrate is determined by measurement of the OD at 400 nm. Therefore, 10 ml cultures containing yeast cells with either pSY1, pUR7034 or pUR2972B were centrifuged, the pellet was resuspended in 4 ml of buffer A (0.1 M NaOAc, pH 5.0 and 1 mM PMSF ), from this 4 ml 500µl was centrifuged again and resuspended in 500 µl PNB-buffer (20 mM Tris-HCl, pH 9.0, 20 mM CaCl2, 25 mM NaCI), centrifuged once again, and finally resuspended in 400µl PNB buffer. This fraction was used to determine the cell bound fraction of lipase.

The remaining 3500µl were spun down, the pellet was resuspended in 4 ml A, to each of this, 40µl laminarinase (ex mollusc, 1.25 mU/µl) was added and first incubated for 3 hours at 37°C, followed by an overnight incubation at 20°C Then the reaction mixture, still containing intact cells, were centrifuged again and the supernatant was used to determined the amount of originally cell wall bound material released through laminarinase incubation. The final pellet was resuspended in 400µl PNP buffer, to calculate the still cell associated part. The blank reaction of a defined volume of specific culture fraction in 4 ml assay buffer was determined, and than the reaction was started through addition of 80µl of substrate solution (100 mM PNP-butyrate in methanol), and the reaction was observed at 25°C at 400 nm in a spectrophotometer.

| | cell bound activity∗ | activity in the medium | laminarinase extract | laminarinase extracted cells | OD660 |
|---|---|---|---|---|---|
| pSY1 | 0.001 (116µl) | 0.001 | 0.028 | 0.000 | 2.6 |
| pUR7034 | 0.293 (220µl) | 0.446 | 0.076 | 0.985 | 2.36 |
| pUR2972B-(1) | 0.494(143µl) | 0.021 | 0.170 | 0.208 | 2.10 |

| | | | | | |
|---|---|---|---|---|---|
| ∗unless otherwise mentioned, the volume of enzyme solution added was 20µl | | | | | |

This result positively demonstrates that a significant amount of lipase activity is immobilized on the surface yeast cell, containing plasmid pUR2972B. Here again, incorporation took place in such a way, that the reaction was catalyzed by cell wall inserted lipase of intact cells, indicated into the exterior orientated immobilization. Furthermore, the release of a significant amount of lipase activity after incubation with laminarinase again demonstrates the presumably covalent incorporation of a heterologous enzyme through gene fusion with the C-terminal part of α-agglutinin.

### 3. Localization

The expression, secretion, and subsequent incorporation of the lipase-α-agglutinin fusion protein into the yeast cell wall was also confirmed through immunofluorescent labelling with anti-lipolase serum essentially as described in EXAMPLE 1, item 2. Localization.

As can be seen in Figure 15, the immunofluorescent stain shows essentially an analogous picture as the α-galactosidase immuno stain, with clearly detectable reactivity on the outside of the cell surface (see Figure 15 A showing a clear halo around the cells and Figure B showing a lighter circle at the surface of the cells), but neither in the medium nor in the interior of the cells. Yeast cells expressing pUR2972B, the *Humicola* lipase-α-agglutinin fusion protein, become homogeneously stained on the surface, indicating the virtually entire immobilization of a chimeric enzyme with an α-agglutinin C-terminus on the exterior of a yeast cell. In the performed control experiment SU10 yeast cells containing plasmid pUR7034 served as a control and here, no cell surface bound reactivity against the applied anti-lipase serum could be detected.

In a similar way variants of *Humicola* lipase, obtained via rDNA techniques, can be linked to the C-terminal part of α-agglutinin, which variants can have a higher stability during (inter)esterification processes.

### EXAMPLE 3 Immobilized Humicola lipase/α-agglutinin on the surface of S. cerevisiae (constitutive expression of immobilized enzyme system)

Plasmid pUR2972 as described in EXAMPLE 2 can be treated with *Eag*I and *Hin*dIII and the about 2.2 kb fragment containing the lipase/α-agglutinin gene can be isolated. Plasmid pSY16 can be restricted with *Eag*I and *Hin*dIII and between these sites the 2.2 kb fragment containing the lipase/α-agglutinin fragment can be ligated resulting in pUR2973. The part of this plasmid that is involved in the production of the chimeric enzyme is similar to pUR2972 with the exception of the signal sequence. Whereas pUR2972 contains the *SUC2*-invertase-signal sequence, pUR2973 contains the α-mating factor signal sequence (see reference 18). Moreover the plasmid pUR2973 contains the *Leu2* marker gene with the complete promoter sequence, instead of the truncated promoter version of pUR2972.

### EXAMPLE 4 Immobilized Geotrichum lipase/α-agglutinin on the surface of S. cerevisiae

The construction and isolation of the 1.4 kb *Nhe*I/*Hind*III fragment comprising the C-terminal part of AGα-1 (α-agglutinin) gene has been described in EXAMPLE 1. For the in-frame gene fusion of the DNA coding for the C-terminal membrane anchor of α-agglutinin to the complete coding sequence of *Geotrichum candidum* lipase B from strain CMICC 335426 (see Figure 8 and SEQ ID NO: 11 and 12), the plasmid pUR2974 can be used. This plasmid, derived from the commercially available pBluescript II SK plasmid, contains the cDNA coding for the complete *G*. *candidum* lipase II on an 1850 bp long *Eco*RI/*Xho*I insert (see Figure 9).

To develop an expression vector for *S. cerevisiae* with homologous signal sequences, the N-terminus of the mature lipase B was determined experimentally by standard techniques. The obtained amino acid sequence of "Gln-Ala-Pro-Thr-Ala-Val..." is in complete agreement with the cleavage site of the signal peptidase on the *G. candidum* lipase II (see reference 19).

For the fusion of the mature lipase B to the *S. cerevisiae* signal sequences of *SUC2* (invertase) or α-mating factor (prepro-αMF) on one hand and the in-frame fusion to the 3' part of the AGα 1 gene PCR technique can be used. The PCR primer lipo3 (see SEQ ID NO: 13) can be constructed in such a way, that the originally present *Eag*I site in the 5'-part of the coding sequence (spanning codons 5-7 of the mature protein) will become inactivated without any alteration in the amino acid sequence. To facilitate the subsequent cloning procedures, the PCR primer can further contain a new *Eag*I site at the 5' end, for the in-frame ligation to *SUC2* signal sequence or prepro-αMF sequence, respectively. The corresponding PCR primer lipo4 (see SEQ ID NO: 16) contains an extra *Nhe*I site behind the nucleotides coding for the C-terminus of lipase B, to ensure the proper fusion to the C-terminal part of α-agglutinin.

PCR oligonucleotides for the in frame linkage of *G. candidum* lipase II to the *SUC2* signal sequence and the C-terminal part of α-agglutinin.

### a: N-terminal transition to either prepro αMF sequence or SUC2signal sequence.

EagI A Q A P R P S L N
primer lipo3: 5'-GGG GCG GCC GCG CAG GCC CCA AGG CGG TCT CTC AAT-3'
lipaseII: 3'-GAC CGG GTC CGG GGT GCC GCC AGA GAG TTA-5'
(non-cod. strand, see SEQ ID NO: 14) )

### b: C-terminal fusion to C part of α-agglutinin

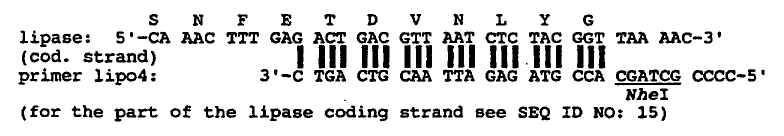
S N F E T D V N L Y G
lipase: 5'-CA AAC TTT GAG ACT GAC GTT AAT CTC TAC GGT TAA AAC-3'
(cod. strand)
primer lipo4: 3'-C TGA CTG CAA TTA GAG ATG CCA CGATCG CCCC-5'
NheI
(for the part of the lipase coding strand see SEQ ID NO: 15)

The PCR product with the modified ends can be generated by standard PCR protocols, using instead of the normal Ampli-*Taq* polymerase the new thermostable VENT polymerase, which also exhibits proofreading activity, to ensure an error-free DNA template. Through digestion of the formerly described plasmid pUR2972 with *Eag*I (complete) and *Nhe*I (partial), the *Humicola* lipase fragment can be exchanged against the DNA fragment coding for lipase B, thereby generating the final *S. cerevisiae* expression vector pUR2975 (see Figure 9).

The *Humicola* lipase-α-agglutinin fusion protein coding sequence can be exchanged against the lipase B/α-agglutinin fusion construct described above by digestion of the described vector pUR2973 with *Eag*I/*Hin*dIII, resulting in pUR2976 (see Figure 9).

### EXAMPLE 5 Immobilized Rhizomucor miehei lipase/α-agglutinin on the surface of S. cerevisiae

The construction and isolation of the 1.4 kb *Nhe*I/*Hin*dIII fragment encoding the C-terminal part of α-agglutinin has been described in EXAMPLE 1. The plasmid pUR2980 contains a 1.25 kb cDNA fragment cloned into the *Sma*I site of commercially available pUC18, which (synthetically synthesizable) fragment encodes the complete coding sequence of triglyceride lipase of *Rhizomucor miehei* (see reference 20), an enzyme used in a number of processes to interesterify triacylglycerols (see reference 21) or to prepare biosurfactants (see reference 22). Beside the 269 codons of the mature lipase molecule, the fragment also harbours codons for the 24 amino acid signal peptide as well as 70 amino acids of the propeptide. PCR can easily be applied to ensure the proper fusion of the gene fragment encoding the mature lipase to the *SUC2* signal sequence or the prepro α-mating factor sequence of *S*. *cerevisiae*, as well as the in-frame fusion to the described *Nhe*I/*Hin*dIII fragment. The following two primers, lipo5 (see SEQ ID NO: 17) and lipo6 (see SEQ ID NO: 20), will generate a 833 bp DNA fragment, which after Proteinase K treatment and digestion with *Eag*I and *Nhe*I can be cloned as an 816 bp long fragment into the *Eag*I/*Nhe*I digested plasmids pUR2972 and pUR2973, respectively (see Figure 7).

EagI A S I D G G I
lipo5: 5'-CCC GCG GCC GCG AGC ATT GAT GGT GGT ATC-3'
lipase (non-cod. strand): 3'-TCG TAA CTA GCA CCA TAG-5'
(for the part of the lipase non-coding strand see SEQ ID NO: 18)

N T G L C T
lipase (cod. strand): 5'-AAC ACA GGC CTC TGT ACT-3'
Lipo6: 3'-TTG TGT CCG GAG ACA TGA CGATCGCGCC-5'
NheI
(for the part of the lipase coding strand see SEQ ID NO: 19)

These new *S. cerevisiae* expression plasmids contain the *GAL7* promoter, the invertase signal sequence (pUR2981) or the prepro-α-mating factor sequence (pUR2982), the chimeric *Rhizomucor miehei* lipase/α-agglutinin gene, the 2 µm sequence, the defective (truncated) *Leu2* promoter and the *Leu2* gene. These plasmids can be transformed into *S. cerevisiae* and grown and analyzed using protocols described in earlier EXAMPLES.

### EXAMPLE 6 Immobilized Aspergillus niger glucose oxidase/GPI anchored cell wall proteins on the surface of S. cerevisiae

Glucose oxidase (β-D:oxygen 1-oxidoreductase, EC 1.1.3.4) from *Aspergillus niger* catalyses the oxidation of β-D-glucose to glucono-δ-lactone and the concomitant reduction of molecular oxygen to hydrogen peroxide. The fungal enzyme consists of a homodimer of molecular weight 150,000 containing two tightly bound FAD co-factors.

Beside the use in glucose detection kits the enzyme is useful as a source of hydrogen peroxide in food preservation. The gene was cloned from both cDNA and genomic libraries, the single open reading frame contains no intervening sequences and encodes a protein of 605 amino acids (see reference 23).

With the help of two proper oligonucleotides the coding part of the sequence is adjusted in a one-step modifying procedure by PCR in such a way that a fusion gene product will be obtained coding for glucose oxidase and the C-terminal cell wall anchor of the *FLO1* gene product or α-agglutinin. Thus, some of the plasmids described in former EXAMPLES can be utilized to integrate the corresponding sequence in-frame between one of the signal sequences used in the EXAMPLES and the *Nhe*I/*Hind*III part of the AGα1 gene.

Since dimerisation of the two monomers might be a prerequisite for activity, in an alternative approach the complete coding sequence for glucose oxidase without the GPI anchor can be expressed in *S. cerevisiae* transformant which already contains the fusion construct. This can be fulfilled by constitutive expression of the fusion construct containing the GPI anchor with the help of the *GAPDH* or *PGK* promoter for example. The unbound not-anchored monomer can be produced by using a DNA construct comprising an inducible promoter, as for instance the *GAL7* promoter.

### EXAMPLE 7 Process to convert raffinose, stachyose and similar sugars in soy extracts with α-galactosidase/α-agglutinin immobilized on yeasts

The yeast transformed with plasmid pUR2969 can be cultivated on large scale. At regular intervals during cultivation the washed cells should be analyzed on the presence of α-galactosidase activity on their surface with methods described in EXAMPLE 1. When both cell density and α-galactosidase activity/biomass reach their maximum, the yeast cells can then be collected by centrifugation and washed. The washed cells can then be added to soy extracts. The final concentration of the yeast cells can vary between 0.1 and 10 g/l, preferably the concentration should be above 1 g/l. The temperature of the soy extract should be < 8 °C to reduce the metabolic activity of the yeast cells. The conversion of raffinose and stachyose can be analyzed with HPLC methods and after 95 % conversion of these sugars the yeasts cells can be removed by centrifugation and their α-galactosidase activity/g biomass can be measured. Centrifugates with a good activity can be used in a subsequent conversion process, whereas centrifugates with an activity of less then 50 % of the original activity can be resuscitated in the growth medium and the cells can be allowed to recover for 2 to 4 hours. Thereafter the cells can be centrifuged, washed and subsequently be used in a subsequent conversion process.

### EXAMPLE 8 Production of biosurfactants using Humicola lipase/α-agglutinin immobilized on yeasts.

The yeast transformed with plasmid pUR2972 or pUR2973 can be cultivated on large scale. At regular intervals during cultivation the washed cells can be analyzed on the presence of lipase activity on their surface with methods described in EXAMPLE 1. When both cell density and lipase/biomass reache their maximum, the yeast cells can be collected by centrifugation and washed. The washed cells can be suspended in a small amount of water and added to a reactor tank containing a mix of fatty acids, preferably of a chain length between 12-18 carbon atoms and sugars, preferably glucose, galactose or sucrose. The total concentration of the water (excluding the water in the yeast cells) might be below 0.1 %. The final concentration of the yeast cells can vary between 0.1 and 10 g/l, preferably the concentration is above 1 g/l. The tank has to be kept under an atmosphere of N₂ and CO₂ in order to avoid oxidation of the (unsaturated) fatty acids and to minimize the metabolic activity of the yeasts. The temperature of mixture in the tank should be between 30-60 °C, depending on type of fatty acid used. The conversion of fatty acids can be analyzed with GLC methods and after 95 % conversion of these fatty acids the yeasts cells can be removed by centrifugation and their lipase activity/g biomass can be measured. Centrifugates with a good activity can be used in a subsequent conversion process, whereas centrifugates with an activity of less then 50 % of the original activity can be resuscitated in the growth medium and the cells can be allowed to recover for 2 to 8 hours. Thereafter the cells can be centrifuged again, washed and used in a subsequent conversion process.

### EXAMPLE 9 Production of special types of triacylglycerols using Rhizomucor miehei lipase/α-agglutinin immobilized on yeasts.

The yeast transformed with plasmid pUR2981 or pUR2982 can be cultivated on a large scale. At regular intervals during cultivation the washed cells can be analyzed on the presence of lipase activity on their surface with methods described in EXAMPLE 1. When both cell density and lipase/biomass reach their maximum, the yeast cells can be collected by centrifugation and washed. The washed cells can be suspended in a small amount of water and can be added to a reactor tank containing a mix of various triacylglycerols and fatty acids. The total concentration of the water (excluding the water in the yeast cells) might be below 0.1 %. The final concentration of the yeast cells can vary between 0.1 and 10 g/l, preferably the concentration is above 1 g/l. The tank has to be kept under an atmosphere of N₂ and CO₂ in order to avoid oxidation of the (unsaturated) fatty acids and to minimize the metabolic activity of the yeasts. The temperature of mixture in the tank should be between 30-70 °C, depending on types of triacylglycerol and fatty acid used. The degree of interesterification can be analyzed with GLC/MS methods and after formation of at least 80 % of the theoretical value of the desired type of triacylglycerol the yeasts cells can be removed by centrifugation and their lipase activity/g biomass can be measured. Centrifugates with a good activity can be used in a subsequent conversion process, whereas centrifugates with an activity of less then 50 % of the original activity is resuscitated in the growth medium and the cells should be allowed to recover 2 to 8 hours. After that the cells can be centrifuged, washed and used in a subsequent interesterification process.

Baker's yeasts of strain MT302/1C, transformed with either plasmid pSY13 or plasmid pUR2969 (described in EXAMPLE 1) were deposited under the Budapest Treaty at the Centraalbureau voor Schimmelcultures (CBS) on 3 July 1992 under provisional numbers 330.92 and 329.92, respectively.

### EXAMPLE 10 Immobilized Humicola lipase/FLO1 fusion on the surface of S. cerevisiae

Flocculation, defined as "the (reversible) aggregation of dispersed yeast cells into flocs" (see reference 24), is the most important feature of yeast strains in industrial fermentations. Beside this it is of principal interest, because it is a property associated with cell wall proteins and it is a quantitative characteristic. One of the genes associated with the flocculation phenotype in *S*. *cerevisiae* is the *FLO1* gene. The gene is located at approximately 24 kb from the right end of chromosome I and the DNA sequence of a clone containing major parts of *FLO1* gene has very recently been determined (see reference 26). The sequence is given in Figure 11 and SEQ ID NO: 21 and 22. The cloned fragment appeared to be approximately 2 kb shorter than the genomic copy as judged from Southern and Northern hybridizations, but encloses both ends of the *FLO1* gene. Analysis of the DNA sequence data indicates that the putative protein contains at the N-terminus a hydrophobic region which confirms a signal sequence for secretion, a hydrophobic C-terminus that might function as a signal for the attachment of a GPI-anchor and many glycosylation sites, especially in the C-terminus, with 46,6 % serine and threonine in the arbitrarily defined C-terminus (aa 271-894). Hence, it is likely that the *FLO1* gene product is localized in an orientated fashion in the yeast cell wall and may be directly involved in the process of interaction with neighbouring cells. The cloned *FLO1* sequence might therefore be suitable for the immobilization of proteins or peptides on the cell surface by a different type of cell wall anchor.

Recombinant DNA constructs can be obtained, for example by utilizing the DNA coding for amino acids 271-894 of the *FLO1* gene product, i.e. polynucleotide 811-2682 of Figure 11. Through application of two PCR primers pcrflol (see SEQ ID NO: 23) and pcrflo2 (see SEQ ID NO: 26) *Nhe*I and *Hin*dIII sites can be introduced at both ends of the DNA fragment. In a second step, the 1.4 kb *Nhe*I/*Hin*dIII fragment present in pUR2972 (either A or B) containing the C-terminal part of α-agglutinin can be replaced by the 1.9 kb DNA fragment coding for the C-terminal part of the FLO1 protein, resulting in plasmid pUR2990 (see Figure 12), comprising a DNA sequence encoding (a) the invertase signal sequence (*SUC2*) preceding (b) the fusion protein consisting of (b.1) the lipase of *Humicola* (see reference 16) followed by (b.2) the C-terminus of FLO1 protein (aa 271-894).

PCR oligonucleotides for the in frame connection of the genes encoding the *Humicola* lipase and the C-terminal part of the *FLO1* gene product.

S N Y A V S T
primer pcrflo1 5'- GAATTC GCT AGC AAT TAT GCT GTC AGT ACC - 3'
NheI
FLO1 gene (non-coding strand) 3'- AGT TTA ATA CGA CAG TCA TGG TGA - 5'
(for the part of the non-coding strand see SEQ ID NO: 24)

FLO1 coding strand 5'-AATAA AATTCGCGTTCTTTTTACG - 3'
primer pcrflo2: 3'-TTAAGCGCAAGAAAAATGC TTCGAACTCGAG - 5'
HindIII
(for the part of the coding strand see SEQ ID NO: 25)

Plasmid pUR2972 (either A or B) can be restricted with *Nhe*I (partial) and *Hin*dIII and the *Nhe*I/*Hind*III fragment comprising the vector backbone and the lipase gene can be ligated to the correspondingly digested PCR product of the plasmid containing the *FLO1* sequence, resulting in plasmid pUR2990, containing the *GAL7* promoter, the *S*. *cerevisiae* invertase signal sequence, the chimeric lipase/*FLO1* gene, the yeast 2 µm sequence, the defective *Leu2* promoter and the *Leu2* gene. This plasmid can be transformed into *S. cerevisiae* and the transformed cells can be cultivated in YP medium including galactose as inductor.

The expression, secretion, localization and activity of the chimeric lipase/FLO1 protein can be analyzed using similar procedures as given in Example 1.

### LITERATURE REFERENCES:

1. Monsan, P., Combes, D. (1988) "Enzyme stabilization by immobilization"; in Meth. in Enzymol. Vol. 137 584-598.
2. Kok, J. (1990) "Genetics of proteolytic systems of lactic acids bacteria" FEMS Microbiol. Rev. 87 15-54.
3. Conzelmann, A., Fankhauser, C., Desponds, C. (1990) "Myoinositol gets incorporated into numerous membrane glycoproteins of *S*. *cerevisiae:* Incorporation is dependent on phosphomannomutase" (SEC53). EMBO 9 653 - 661.
4. Lipke, P.,N., Wojciechowicz, D., Kurjan, J. (1989) "AGα1 is the structural gene for the *Saccharomyces cerevisiae* α-agglutinin, a cell surface glycoprotein involved in cell-cell interactions during mating" Mol. Cell. Biol. 9 3155-3165.
5. Roy, A., Lu, C.F., Marykwas,D., Lipke, P., Kurja, J. (1991) "The AGA1 gene product is involved in cell surface attachment of the *S*. *cerevisiae* cell adhesion glycoprotein a-agglutinin", Mol. Cell. Biol. 11 4196-4206.
6. Teunissen, A.W.R.H., van den Berg, J.A., Steensma, H.Y. (1993) "Physical localization of the flocculation gene *FLO1* on chromosome I of *S. cerevisiae,* Yeast 9 (1) 1-10.
7. Yanisch Perron, C., Viera, J., Messing, J. (1985) "Improved M13 phage cloning vectors and host strains: nucleotide sequence of the M13 mp18 and pUC19 vectors." Gene 33 103-119.
8. Chung, C. T., Niemela, S. L., Miller, R. H. (1989) "One step preparation of competent *E*. *coli:* Transformation and storage of bacterial cells in the same solution" Proc. Natl. Acad. Sci. USA 86 2172-2175.
9. Sanger, F., Nicklen, S., Coulson, A. R. (1977) "DNA sequencing with chain terminating inhibitors" Proc. Natl. Acad. Sci. USA 74 5463-5467.
10. Hsiao, K. (1991) "A fast and simple procedure for sequencing double stranded DNA with Sequenase" Nucl. Acids Res. 19 2787.
11. Klebe, RJJ., Harriss, V., Sharp, Z.D., Douglas, M.G. (1983) "A general method for polyethylene glycol induces genetic transformation of bacteria and yeast" Gene 25 333-341.
12. Overbeeke, N., Fellinger, A. J., Toonen, M. Y., van Wassenaar, P. D., Verrips, C. T. (1989) "Cloning and nucleotide sequence of the α-galactosidase gene from *Cyamopsis tetragonoloba"* Plant Mol. Biol. 13 541-550.
13. Laemmli, U. K. (1970) "Cleavage of structural proteins during the assembly of heads of bacteriophage T4." Nature 227 680-685.
14. Towbin, H. Steahelin, T., Gordon, J. (1979) "Electrophoretic transfer of proteins from polyacrylamide gels to nitrocellulose sheets: Procedure and some applications" Proc. Natl. Acad. Sci. USA 76 4350-4354.
15. Watzele, M., Klis, F., Tanner, W. (1988) "The immunological and molecular characterization of α-agglutinin from S. *cerevisiae*" EMBO J. 7 1483-1488
16. Boel, E., Huge-Jensen, B., Brown, J. D. (1989) *"Humicola* lipase and process for the production of recombinant *Humicola* lipases" EP-A1-0 305 216.
17. Verbakel, J.M.A. (1991) "Heterologous gene expression in the yeast *Saccharomyces cerevisiae"* PhD thesis, Rijksuniversiteit Utrecht, The Netherlands
18. Kurjan, J., Herskowitz, I. (1982) "Structure of a yeast Pheromone Gene (MFα): A putative α-Factor precursor contains four tandem copiers of mature α-factor" Cell 30 933-943.
19. Shimada, Y., Sugihara, A., Tominaga, Y., lizumi, T., Tsunasawa, S. (1989) "cDNA molecular cloning of Geotrichum candidum lipase" J. Biochem, 106 383-388.
20. Boel, E., Huge-Jensen, B., Christensen, M., Thim, L, Fiil, N. (1988) "Rhizomucor miehei Triglyceride Lipase is Synthesized as a Precursor" Lipids, Vol.23, No 7, 701-706.
21. Schuch, R., Mukherjee, K.D. (1988) in "World conference on Biotechnology for the fat and oil industry" ISBN 0-935315-21-7, 328-329.
22. Kosaric, N., Cairns, W.L, Gray, N.C.C. (editors) (1987) "Biosurfactants and Biotechnology" Marcel Dekker Inc., New York, Vol. 25.
23. Frederick, K.R., Tung, J., Emerik, R.S., Masiarz, F.R., Chamberlain, S.H., Vasavada, A., Rosenberg, S. (1990) "Glucose oxidase from Aspergillus niger". J. Biol. Chem., Vol.265, No.7, 3793-3802.
24. Johnston, J.R., Reader, H.P. (1983) "Genetic control of flocculation" in 'Yeast Genetics, Fundamental and applied aspects', Spencer, J.F.T. (Editor), ISBN 0-540-90793-9, p. 205-224.
25. Schreuder, M.P., Brekelmans, S., Van den Ende, H., Klis, F.M. (1993) "Targeting of a Heterologous Protein to the Cell Wall of *Saccharomyces cerevisiae"* Yeast 9 399-409
26. Teunissen, A.W.R.H., Holub, E., Van der Hucht, J., Van Den Berg, J.A., Steensma, H.Y. (1993) "Sequence of the Open reading frame of the *FLO1* Gene from *Saccharomyces cerevisiae"* YEAST 9 423-427.
27. Harmsen, M.M., Langedijk, A.C., Van Tuinen, E., Geerse, R.H.; Raue, H.A., Maat, J. (1993) Effect of a *pmr1* disruption and different signal sequences on the intracellular processing and secretion of *Cyamopsis tetragonoloba* α-galactosidase by *Saccharomyces cerevisiae* Gene 125 115-123

## Claims

1. A method for immobilizing an enzyme, comprising the use of recombinant DNA techniques for producing an enzyme or a functional part thereof linked to the cell wall of a host cell, preferably a microbial cell, and whereby the enzyme or functional fragment thereof is localized at the exterior of the cell wall.

2. The method of claim 1, wherein the enzyme or the functional part thereof is immobilized by linking to the C-terminal part of a protein that ensures anchoring in the cell wall.

3. A recombinant polynucleotide comprising a structural gene encoding a protein providing catalytic activity and at least a part of a gene encoding a protein capable of anchoring in a eukaryotic or prokaryotic cell wall, said part encoding at least the C-terminal part of said anchoring protein.

4. The polynucleotide of claim 3, further comprising a sequence encoding a signal peptide ensuring secretion of the expression product of the polynucleotide.

5. The polynucleotide of claim 4, wherein the signal peptide is derived from a protein selected from the group consisting of glycosyl-phosphatidyl-inositol (GPI) anchoring protein, α-factor, α-agglutinin, invertase or inulinase, α-amylase of *Bacillus*, and proteinases of lactic acid bacteria.

6. The polynucleotide of any of claims 3-5, wherein the protein capable of anchoring in the cell wall is selected from the group consisting of α-agglutinin, AGA1, FLO1, Major Cell Wall Protein of lower eukaryotes, and proteinases of lactic acid bacteria.

7. The polynucleotide of any of claims 3-6, operably linked to a promoter, preferably an inducible promoter.

8. The polynucleotide of any of claims 3-7, wherein the protein providing catalytic activity is a hydrolytic enzyme, e.g. a lipase.

9. The polynucleotide of any of claims 3-7, wherein the protein providing catalytic activity is an oxidoreductase, e.g. an oxidase.

10. A recombinant vector comprising a polynucleotide as claimed in any of claims 3-9.

11. The recombinant vector of claim 10, wherein the protein providing catalytic activity exhibits said catalytic activity when present in a multimeric form, said vector further comprising a second polynucleotide comprising a structural gene encoding the same protein providing catalytic activity combined with a sequence encoding a signal peptide ensuring secretion of the expression product of said second polynucleotide, said second polynucleotide being operably linked to a regulatable promoter, preferably an inducible or repressible promoter.

12. A chimeric protein encoded by a polynucleotide as claimed in any of claims 3-9.

13. A host cell, preferably a microorganism, containing a polynucleotide as claimed in any of claims 3-9 or a vector as claimed in claim 10 or 11.

14. A host cell, preferably a microorganism, containing a polynucleotide as claimed in any of claims 3-9 or a vector as claimed in claim 10, wherein the protein providing catalytic activity exhibits said catalytic activity when present in a multimeric form, said microorganism further comprising a second polynucleotide comprising a structural gene encoding the same protein providing catalytic activity combined with a sequence encoding a signal peptide ensuring secretion of the expression product of said second polynucleotide, said second polynucleotide being operably linked to a regulatable promoter, preferably an inducible or repressible promoter and said second polynucleotide being present either in another vector or in the chromosome of said microorganism.

15. The host cell or microorganism of claim 13 or 14, having at least one of said polynucleotides integrated in its chromosome.

16. A host cell, preferably a microorganism, having a protein as claimed in claim 12 immobilized on its cell wall.

17. The host cell or microorganism of any of claims 13-16, which is a lower eukaryote, in particular a yeast.

18. A process for carrying out an enzymatic process by using an immobilized catalytically active protein, wherein a substrate for said catalytically active protein is contacted with a host cell or microorganism as claimed in any of claims 13-17.
